(19) Europäisches Patentamt
European Patent Office
Office européen des brevets

(11) EP 3 962 491 B1

(12) EUROPEAN PATENT SPECIFICATION

(45) Date of publication and mention of the grant of the patent:
**06.08.2025 Bulletin 2025/32**

(21) Application number: **20722582.2**

(22) Date of filing: **30.04.2020**

(51) International Patent Classification (IPC):
***A61K 31/60*** *(2006.01)* ***A61K 39/00*** *(2006.01)*

(52) Cooperative Patent Classification (CPC):
(C-Sets available)
**C07K 16/32; A61K 31/337; A61K 39/001164; A61K 39/39558; C07K 16/30;** A61K 2039/55555; B82Y 5/00; C07K 2317/34; C07K 2317/77; C07K 2317/92; C07K 2317/94 (Cont.)

(86) International application number:
**PCT/EP2020/062002**

(87) International publication number:
**WO 2020/221849 (05.11.2020 Gazette 2020/45)**

(54) **INTRACELLULAR DELIVERY OF ANTI-KRAS ANTIBODIES FORMULATED INTO NANOCAPSULES**

INTRAZELLULÄRE ABGABE VON IN NANOKAPSELN FORMULIERTEN ANTI-KRAS-ANTIKÖRPERN

ADMINISTRATION INTRACELLULAIRE D’ANTICORPS ANTI-KRAS FORMULÉS DANS DES NANOCAPSULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **01.05.2019 EP 19172120**
**19.03.2020 EP 20382206**

(43) Date of publication of application:
**09.03.2022 Bulletin 2022/10**

(73) Proprietor: **Universidade De Santiago De Compostela**
**15782 Santiago de Compostela (ES)**

(72) Inventors:
- **ALONSO FERNANDEZ, María José**
**15782 Santiago de Compostela (ES)**
- **TEIJEIRO OSORIO, Desireé**
**15782 Santiago de Compostela (ES)**

(74) Representative: **ZBM Patents - Zea, Barlocci & Markvardsen**
**Rambla de Catalunya, 123**
**08008 Barcelona (ES)**

(56) References cited:
**EP-A1- 3 173 099** **EP-A1- 3 173 428**
**WO-A2-2012/047317** **WO-A2-2018/069871**

- **SEUNG-MIN SHIN ET AL: "Antibody targeting intracellular oncogenic Ras mutants exerts anti-tumour effects after systemic administration", NATURE COMMUNICATIONS, vol. 8, 10 May 2017 (2017-05-10), pages 1 - 14, XP055434123, DOI: 10.1038/ncomms15090**
- **KI JUNG LIM ET AL: "A Cancer Specific Cell-Penetrating Peptide, BR2, for the Efficient Delivery of an scFv into Cancer Cells", PLOS ONE, vol. 8, no. 6, 1 June 2013 (2013-06-01), pages e66084, XP055700393, DOI: 10.1371/journal.pone.0066084**

Remarks:
The complete document including Reference Table(s) and the Sequence Listing(s) can be downloaded from the EPO website

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

(52) Cooperative Patent Classification (CPC): (Cont.)

C-Sets
**A61K 31/337, A61K 2300/00**

## Description

### FIELD OF THE INVENTION

The present invention relates to intracellular delivery of antibodies against mutated KRAS oncoproteins where the antibodies are formulated into particles, nanocapsules or other nanoentities for making them able to access the inside of cells where they release their contents.

### BACKGROUND OF THE INVENTION

Targeted delivery of pharmaceutical agents into the body has been an ongoing challenge. For example, many drugs cannot effectively exert their action because of their difficult access to target cells. So far monoclonal antibodies-based cancer therapies could only be directed towards extracellular proteins, whereas hundreds of intracellular oncoproteins of extraordinary relevance in cancer remain undruggable.

Mutationally activated RAS genes (HRAS, KRAS, and NRAS) comprise the most frequently mutated gene family in cancer. The three RAS genes encode four 188-189 amino acid proteins that share 82%-90% amino acid sequence identity. KRAS is a gene that acts as an on/off switch in cell signaling. When it functions normally, it controls cell proliferation. When it is mutated, negative signaling is disrupted and cells can continuously proliferate, and often develop into cancer. The KRAS protein is a GTPase and is usually connected to cell membranes because of the presence of an isoprene group on its C-terminus. In normal quiescent cells, RAS is predominantly GDP-bound and inactive. Upon extracellular stimuli, activation of receptor tyrosine kinases (RTKs) and other cell-surface receptors, there is rapid and transient formation of RAS-GTP, leading to engagement of effector proteins that then regulate a diversity of intracellular signaling networks and thereby control mitogenic processes.

Cancer-associated RAS genes are characterized by mutations that encode single amino acid substitutions primarily at residues glycine-12 (G12), glycine-13 (G13), or glutamine-61 (Q61). These mutations make RAS GTP-bound and constitutively active independent of extracellular stimuli, resulting in overstimulation of signaling pathways leading to cancer growth.

Mutationally activated RAS genes were first identified in human cancer cells by 1982. More than 30 years later, intensive sequencing of the cancer genome has revealed that, despite the identification of over 500 validated cancer genes (COSMIC database, catalogue of somatic mutations in cancer), the three RAS genes (HRAS, NRAS and KRAS) still constitute the most frequently mutated oncogene family in human cancers (Table 1), being detected in 25-30% of all tumor samples sequenced (Cox et al., 2014).

Table 1. Frequency of RAS mutations in human cancers (Cox et al., 2014)

| **Cancer** | ***% KRAS*** | ***% NRAS*** | ***% HRAS*** | **% All *RAS*** |
|---|---:|---:|---:|---:|
| Pancreatic ductal adenocarcinoma | 97.7 | 0.0 | 0.0 | 97.7 |
| Colorectal adenocarcinoma | 44.7 | 7.5 | 0.0 | 52.2 |
| Multiple myeloma | 22.8 | 19.9 | 0.0 | 42.6 |
| Lung adenocarcinoma | 30.9 | 0.9 | 0.3 | 32.2 |
| Skin cutaneous melanoma | 0.8 | 27.6 | 1.0 | 29.4 |
| Uterine corpus endometrioid carcinoma | 21.4 | 3.6 | 0.4 | 24.6 |
| **Uterine carcinosarcoma** | 12.3 | 1.8 | 0.0 | 14.0 |
| Thyroid carcinoma | 1.0 | 8.5 | 3.5 | 13.0 |
| Stomach adenocarcinoma | 11.4 | 0.9 | 0.0 | 12.3 |
| Acute myeloid leukaemia | 3.1 | 6.7 | 1.6 | 11.4 |
| Bladder urothelial carcinoma | 3.1 | 1.4 | 5.9 | 10.6 |
| Cervical adenocarcinoma | 8.3 | 0.0 | 0.0 | 8.3 |
| Head and neck squamous cell carcinoma | 0.5 | 0.3 | 4.7 | 5.5 |
| Gastric carcinoma | 4.0 | 1.0 | 0.0 | 5.0 |
| Esophageal adenocarcinoma | 4.1 | 0.0 | 0.7 | 4.8 |

Even though all are expressed widely in adult tissues and in tumors, KRAS mutation is much more frequent in human cancer than NRAS or HRAS, with KRAS comprising 85% of all RAS mutations in cancer, followed by NRAS (12%) and, infrequently, HRAS (3%). One explanation for the high frequency of KRAS mutations, relative to NRAS and HRAS, is that KRAS protein has unique properties that favor oncogenesis.

Thus, for example, in pancreatic ductal adenocarcinoma (PDAC, 90% of all pancreatic cancers) and lung adenocarcinoma (LAC, 40% of all lung cancers) there is a near 100% and 25-30% frequency of KRAS mutations, respectively. In colorectal carcinomas (CRC), KRAS is also the predominant mutated isoform (45%) (Liu et al., 2019; Ferrer et al., 2018; Cox et al., 2014).

There are also cancer-type differences in the relative frequency of mutations at positions G12, G13 or Q61. In PDAC, LAC and CRC, KRAS mutations are found predominantly at G12. There are also cancer-type differences in the substitutions seen at a given residue. For example, at G12, in PDAC and CRC the predominant substitution is G12D, followed by G12V. In contrast, in LAC, the major substitution is G12C, which is rare in PDAC. Genetics experiments have demonstrated the dependency of pancreatic and lung adenocarcinoma to oncogenic KRAS expression (Fisher et al., 2001; Ying et al., 2012), highlighting its value as a molecular target. Therefore, KRAS proteins are exceptionally important players in cancer and represent an extraordinarily well-validated drug target.

Decades of efforts have been oriented to develop small molecules targeting oncogenic KRAS. However, direct inhibition of KRAS mutants has proven extremely challenging, mainly due to difficulties in identifying druggable pockets for small molecule binding on the surface of RAS, and no agents have been clinically approved to date. This has been attributed to the "smoothness" of the protein's surface, with the exception of a GTP-binding pocket that holds on to its substrate too tightly to be displaced.

For the treatment of cancers harboring KRAS mutations, various strategies have been proposed, for example:

(1) inhibition of expression of the RAS protein by the hammer head ribozyme or antisense oligonucleotide;
(2) prevention of membrane localization of RAS by farnesyl-transferase inhibitors or acyl protein thioesterase 1 (APT1) inhibitors;
(3) blocking downstream effectors of the RAS signal pathway (inhibition of Raf or MEK via antisense oligonucleotide or chemical inhibitors); and
(4) silencing of a mutant KRAS transcript by RNA interference that distinguishes one nucleotide difference between wild-type and mutant KRAS.

However, most of these strategies had limitations, such as non-selectivity or low therapeutic efficacy. Therefore, a more effective strategy harnessed with mutant KRAS selectivity is required to treat mutant KRAS-related cancers.

Antibodies, with large surface area paratopes, are excellent to specifically target proteins with high affinity. However, so far antibodies-based cancer therapies have only been directed mainly towards extracellular proteins, whereas hundreds of intracellular oncoproteins of extraordinary relevance in cancer remain undruggable. Attempts are being directed towards the modification of antibodies to confer them the capacity to cross the cell membrane by using two main approaches: (i) chemical conjugation with cell penetrating peptides (CPPs); and (ii) fusion with part of internalizing autoantibodies responsible for their intrinsic ability to enter cells.

However, in most cases the antibody concentrations used *in vitro* are in the micromolar range (Shin et al., 2017; Akishiba et al., 2017) and, in those studies reporting *in vivo* data, the dosage regimens are not clinically translatable (Shin et al., 2017). In the case of CPP-based strategies, main limitations are related with factors such as a poor stability *in vivo,* the lack of tissue/cellular specificity, a non-efficient release from intracellular endocytic vesicles and the delay in binding to the target due to the presence of the CPP. Some drawbacks of CPP-based strategies could be circumvented by using the aforementioned fusion protein approaches. However, fusion protein generation is relatively tedious and is not amenable for rapid screening (Slastnikova et al., 2018; Singh et al., 2019).

Thus, there is a need for novel intracellular delivery systems that can deliver clinically relevant antibody doses *in vivo* and work as versatile, cost-effective, platforms for intracellular antibody-based therapeutics discovery and development.

As far as we know, in the case of anti-KRAS antibodies they have mainly been described in relation to diagnostic uses and as research tools for studying the RAS polypeptides. However, Shin et al., 2017 reported the development of a human IgG1 format antibody, RT11, which internalizes into the cytosol of living cells and selectively binds to the activated GTP-bound form of various oncogenic Ras mutants to block the interactions with effector proteins, thereby suppressing downstream signaling and exerting anti-proliferative effects in a variety of tumor cells harboring oncogenic Ras mutants. The RT11 antibody was generated by integrating the Ras GTP-specific binding VH domain with a cytosol-penetrating VL fragment to form a single IgG format antibody. Thus, this mechanism is different from the herein described encapsulation of anti-KRAS antibodies.

Although several companies and scientific papers claim the importance of developing monoclonal antibodies to target intracellular proteins, as far as we know there is not a single disclosure of *in vivo* data showing the efficacy of a

nanotechnology-based approach for the intracellular delivery of therapeutic antibodies after systemic IV administration. Thus, improvements in the delivery of pharmaceutical agents are needed.

## SUMMARY OF THE INVENTION

One problem to be solved by the present invention is the intracellular delivery of anti-KRAS antibodies in clinically relevant concentrations so that they lead to a therapeutic effect.

The solution is based on the finding by the present inventors that anti-KRAS antibodies formulated into particular nanoentities are able to be intracellular delivered and further, are able to perform their biological activity inside the cell.

As discussed in the working Examples herein, the inventors have successfully associated anti-KRAS monoclonal antibodies to different polymeric nanocapsule compositions. As shown in Example 5 such nanocapsules are capable of penetrating cell membranes and deliver the antibody to the inside of the cell membrane. Nanocapsules containing anti-KRAS G12V mAbs efficiently internalized in colon adenocarcinoma cells and the internalized antibody showed a preferential localization at the target site, i.e., at the inner side of the plasma membrane, where oncogenic KRAS is localized. Similarly, in Example 6, anti-KRAS G12V mAb-loaded polymeric nanocapsules efficiently internalized in lung adenocarcinoma cells. Further, in Example 5 it is demonstrated that such nanocapsules are able to inhibit the *in vitro* growth of KRAS G12V mutant tumor cells in percentages from 25% to 50%. Thus, it is demonstrated that the anti-KRAS antibodies formulated into nanocapsules are able to be intracellular delivered and further, are able to perform their biological activity inside the cell.

Remarkably, Example 7 provides the first *in vivo* evidence of the efficacy of nanocapsules containing anti-KRAS G12V mAbs in significantly reducing tumor growth in two mice models: pancreatic xenograft and colon orthotopic tumor models without showing signs of treatment toxicity.

As discussed, anti-KRAS antibodies as such are unable to cross cell membranes and are unable to interact with the KRAS polypeptides, and thereby blocking the function of the corresponding intracellular target proteins and eliciting a therapeutic response. The inventors have demonstrated that encapsulation of anti-KRAS antibodies in polymeric nanocapsules is an effective approach to deliver these antibodies directly into the cytosol of living cells, thus eliciting a response in both *in vitro* and *in vivo* studies.

As said before, despite many attempts in developing monoclonal antibodies to target intracellular proteins, as far as we know there is not a single disclosure of *in vivo* data showing the efficacy of a nanotechnology-based delivery system for intracellular localization after IV administration. It is thus believed that the present invention provides the first approach for intracellular delivery of encapsulated anti-KRAS antibodies with successful results *in vivo*.

Therefore, the invention solves a technical problem which the skilled in the art have been attempting to solve for a long time, and also fulfills a long-felt need.

As discussed herein, the fact that present nanocapsules are capable of penetrating cell membranes and deliver the antibody at relevant clinical doses to the inside of the cell membrane and escape the lysosomal compartment is a significant advantage in general.

For instance, in relation to a particular embodiment herein where nanocapsules also comprises a cell-penetrating peptide and/or a tumor/tissue-penetrating peptide one may even obtain a higher dose and better localization of the antibody into the tumoral tissue and into the cancer cells.

The invention is set out in the appended set of claims.

Accordingly, a first aspect of the present invention relates to a composition, comprising a plurality of nanoentities comprising an inner core surrounded by an outer shell, the outer shell comprising a polymer, the inner core comprising at least one hydrophobic compound, wherein the nanoentities comprise a pharmaceutical agent, wherein the pharmaceutical agent is an antibody or a fragment thereof, wherein the nanoentities are capable of intracellularly delivering the antibody or the fragment thereof, and wherein the antibody or the fragment thereof binds to an epitope of an activated mutated KRAS protein.

A second aspect of the present invention relates to a method for preparing a composition according to the first aspect, comprising the steps of:

a) preparing an aqueous phase comprising a polymer;
b) preparing a hydrophobic phase comprising a hydrophobic compound;
c) adding an antibody or a fragment thereof which binds to an intracellular epitope of an activated mutated KRAS protein to the aqueous phase or, optionally to the hydrophobic phase if the antibody or the fragment is highly concentrated; and
d) mixing the aqueous phase and the hydrophobic phase.

A third aspect of the present invention relates to a composition for use as a medicament.

A fourth aspect of the present invention relates to a composition for use in treating or preventing a disease

associated with a mutation in a KRAS gene.

## DESCRIPTION OF THE DRAWINGS

FIG. 1: This figure illustrates the stability of anti-KRAS G12V mAb-loaded HA nanocapsules (NCs) (HA 290 kDa NCs; 0.5 mg/mL mAb) in human plasma measured by Dynamic Light Scattering (DLS).

FIG. 2: This figure illustrates the stability of anti-KRAS G12V mAb-loaded HA nanocapsules (NCs) (HA 290 kDa NCs; 0.5 mg/mL mAb) in human plasma measured by Nanoparticle Tracking Analysis (NTA).

FIG. 3: This figure illustrates the cell internalization of anti-KRAS G12V mAb (labeled with Alexa Fluor® 488) elicited by HA nanocapsules (NCs) in SW480 colorectal adenocarcinoma cells expressing KRAS G12V mutation by imaging flow Cytometry. The effective internalization was determined by labeling cytoplasm acidic organelles of living cells with a Lysotracker® fluorescent marker.

FIG. 4: This figure shows (A) inhibition of cell proliferation and (B) reduction of ERK phosphorylation produced after incubation of H441 adenocarcinoma lung cancer cells expressing KRAS G12V mutation with C16-HA nanocapsules (NCs) (blank and anti-KRAS G12V-loaded NCs at 166 nM mAb, 0.4 ng mAb/cell dose). [C(-): untreated cells; BL: blank C16-HA NCs; aG12V NC: anti-KRAS G12V mAb-loaded C16-HA NCs].

FIG. 5: This figure shows the reduction in tumor growth in mice treated with anti-KRAS G12V mAb-loaded PSA-tLyp-1 nanocapsules (NCs) during the treatment period compared to the control group. [C (-): saline; aG12V NC: anti-KRAS loaded tLyp1-PSA NCs; IP administration; Pancreatic subcutaneous xenograft tumor model from PA-TU-8902 cells expressing KRAS G12V mutation].

FIG. 6: This figure shows the reduction in final tumor weight of mice treated with anti-KRAS G12V mAb-loaded HA NCs during 3 weeks compared to the control group. [C (-): PBS; aG12V NC: anti-KRAS loaded HA NCs; IV administration; Colorectal orthotopic tumor model from SW480 cell line-derived subcutaneous tumor fragments bearing KRAS G12V mutation].

FIG. 7: This figure illustrates the remarkable histologic regression changes in tumors of mice treated with anti-KRAS G12V mAb-loaded HA NCs during 3 weeks compared to the control group. Tumor slides were scanned and analyzed with the NDP-View2 software (Hamamatsu), and the percentage of necrosis was calculated in for each slide. [C (-): PBS; aG12V NC: anti-KRAS loaded HA NCs; IV administration; Colorectal orthotopic tumor model from SW480 cell line-derived subcutaneous tumor fragments bearing KRAS G12V mutation].

FIG. 8: This figure shows the body weight changes of mice treated with anti-KRAS G12V mAb-loaded HA NCs compared to the control group over the 3 weeks treatment period [C (-): PBS; aG12V NC: anti-KRAS loaded HA NCs; Colorectal orthotopic tumor model from SW480 cell line-derived subcutaneous tumor fragments bearing KRAS G12V mutation].

## DETAILED DESCRIPTION OF THE INVENTION

The present invention relates to a composition comprising particles, including nanocapsules or other nanoentities comprising an inner core surrounded by an outer shell, where the inner core comprises a hydrophobic compound such as an oil and the outer shell comprises a polymer such as polysialic acid (PSA), hyaluronic acid, polyglutamic acid, polyaspartic acid, polymalic acid or polylactic acid. The nanoentities further comprise a pharmaceutical compound that is an antibody or a fragment thereof against an intracellular located target wherein the antibody or the fragment thereof binds to an epitope of an activated mutated KRAS protein. The particles of the present invention are able to access the inside of the cells where they release the antibody. Moreover, the polymer coating of the oily core may confer greater stability and protection against aggregation, a change in the drug release profile of the associated antibody, an increased cellular internalization and specific interaction with certain cell types.

The term "inner core" may herein alternatively be termed "inner portion" and these terms may be used interchangeably herein.

Antibodies against mutated KRAS polypeptides

Some of the first antibodies able to bind to mutated KRAS polypeptides are described in US5084380 that discloses monoclonal antibodies that react with activated KRAS polypeptides containing amino acid mutations at position 12 of the KRAS polypeptide and that do not react with proteins containing the normal amino acid glycine at position 12. The inventor of US5084380 identified antibodies E184 and E170 that reacted with KRAS polypeptides containing glutamic acid at position 12 instead of glycine (G12E), R256 that reacted with a KRAS polypeptide containing arginine at position 12 instead of glycine (G12R) and DWP that reacted with an KRAS polypeptide containing valine at position 12 instead of glycine (G12V).

The monoclonal antibodies E170 and E184 were raised against a synthetic peptide corresponding to amino acids 5-16 of a mutated ras gene encoding glutamic acid instead of glycine at position 12. These antibodies show specificity for dodecapeptides containing glutamic acid at position 12 but not with dodecapeptides containing Gly, Asp, Ser, Arg, Cys, Ala or Val at position 12.

Monoclonal antibody R256 was raised against a synthetic peptide corresponding to amino acids 5-16 of a mutated ras gene encoding arginine instead of glycine at position 12. Monoclonal antibody R256 specifically reacted with dodecapeptides containing Arg at position 12 but did not react with dodecapeptides containing Gly, Glu, Asp, Ser, Cys, Val or Ala at position 12.

DWP was raised against a synthetic peptide corresponding to amino acids 5-16 of a mutated ras gene encoding valine instead of glycine at position 12. DWP reacted in competition assays with peptides containing Val or Cys at position 12 but not with peptides containing Gly, Arg, Ser, Ala, Asp or Glu at position 12 (Carney et al., 1986 and EP0190033).

The hybidroma cell lines which was found to secrete the monoclonal antibodies were deposited in the American Type Tissue Culture Collection (ATCC) under the Budapest Treaty and E184 was given accession number HB9194, E170 accession number HB9195, R256 accession number HB9196 and DWP accession number HB8698.

In particular embodiments of the present invention, the antibody is one of the antibodies described above disclosed in US5084380 or a fragment thereof.

It is further described in US5443956, that the monoclonal antibodies reactive with mutated ras p21 proteins having aspartic acid at position 12 (G12D) were produced by hybridoma cell lines D113, D205, and D210. These cell lines were deposited with the ATCC under the Budapest Treaty. Hybridoma cell line D113 was accorded ATCC designation number HB10086. Hybridoma cell line D205 was accorded ATCC designation number HB10061. Hybridoma cell line D210 was accorded ATCC designation number HB10083.

In particular embodiments of the present invention, the antibody is one of the antibodies described above disclosed in US5443956 or a fragment thereof.

Other monoclonal antibodies binding to mutated KRAS polypeptides have been developed, for example, by Abcam and include ab221163, ab264094 and ab264095; by NewEast Bioscience and include Catalog Number: 26036 (G12D), 26038 (G12D), 26193 (Q61L), 26474 (G13R), 26192 (Q61R), 26477 (G13V), 26195 (Q61H), 26191 (G13A) and 26186 (G12S); by Cell Signaling and include Ras (G12D Mutant Specific) (D8H7) Rabbit mAb #14429 and Ras (G12V Mutant Specific) (D2H12) Rabbit mAb #14412; and by Sigma-Aldrich and include G12V Ref. OP38 Anti-Pan-Ras (Ab-1) Mouse mAb.

WO2012047317 discloses compositions comprising antibody, TAB-004 and antibody/nanoparticle conjugates

In one embodiment, the epitope of the activated mutated KRAS protein comprises a mutation of the glycine residue at position 12, the glycine residue at position 13 or the glutamine residue at position 61 of the amino acid sequence of SEQ ID NO: 39 that corresponds to the polypeptide sequence of the human Isoform 2B of GTPase KRas (the major isoform expressed in human tumors) having UNIPROT reference P01116-2 (RASK_HUMAN).

In another embodiment, the mutation at position 12 of the amino acid sequence of SEQ ID NO: 39 is selected from the group consisting of arginine, G12R, aspartic acid, G12D, valine G12V and cysteine G12C. In a further embodiment the mutation at position 13 of the amino acid sequence of SEQ ID NO: 39 is selected from the group consisting of arginine, G13R, aspartic acid, G13D and valine G13V. In a still further embodiment, the mutation at position 61 of the amino acid sequence of SEQ ID NO: 39 is selected from the group consisting of glutamine Q61R, leucine Q61L and histidine Q61H.

The skilled person knows how to select antibodies that binds to such described epitopes however, in one embodiment, the antibody or the fragment thereof binds to an epitope of a sequence selected from the group consisting of KLVVVGAVGVGK SEQ ID NO: 40, KLVVVGADGVGK SEQ ID NO: 41, and KLVVVGACGVGK SEQ ID NO: 42.

In a further embodiment, the antibody or the fragment thereof binds to the same epitope of the human activated mutated KRAS protein as the antibody obtainable from a hybridoma cell line with deposit number selected from the group consisting of ATCC-HB-10086 D113, ATCC-HB-10083 D210 and ATCC-HB-8698 DWP.

In a still further embodiment, the antibody or the fragment thereof is obtainable from a hybridoma cell line with deposit number selected from the group consisting of ATCC-HB-10086 D113, ATCC-HB-10083 D210 and ATCC-HB-8698 DWP.

The above-mentioned hybridomas were obtained from ATCC, revitalized and the corresponding antibodies were

obtained.

In a further embodiment, the antibody or the fragment thereof binds to the same epitope of the human activated mutated KRAS protein as the antibody obtainable from a hybridoma cell line with deposit number selected from the group consisting of DSM ACC3358, ATCC-HB-10083 D210, ATCC-HB-8698 DWP, ATCC-HB-10086-D113 and DSM ACC3359.

In a still further embodiment, the antibody or the fragment thereof is obtainable from a hybridoma cell line with deposit number selected from the group consisting of DSM ACC3358, ATCC-HB-10083 D210, ATCC-HB-8698 DWP, ATCC-HB-10086-D113 and DSM ACC3359.

Hybridoma cell lines DSM ACC3358 and DSM ACC3359 were deposited in the Leibniz Institute DSMZ-German Collection of Microorganisms and Cell Cultures GmbH (Inhoffenstraße 7B. 38124 Braunschweig. GERMANY) on the deposition date October 16th, 2019. The deposited cells are viable and keep all their features related to their deposit. Mouse hybridoma cell line G12D D113-2-1 was deposited under the accession number DSM ACC3358 and mouse hybridoma cell line G12V DWP-1-1-3 under the accession number DSM ACC3359. The depositor is Universidade de Santiago de Compostela, Spain.

The DNA sequence of the anti-KRAS antibody from the hybridoma cell line deposited as DSM ACC3359 was obtained. The corresponding antibody is a G12V anti-KRAS monoclonal antibody. In one embodiment, the antibody or the fragment thereof comprises a heavy-chain variable region CDR1: (SEQ ID NO: 33) SGYYWN, a heavy-chain variable region CDR2: (SEQ ID NO: 34) YIGYDGTNNYNPSLKN, a heavy-chain variable region CDR3: (SEQ ID NO: 35) LWDY, a light-chain variable region CDR1: (SEQ ID NO: 36) RSSQTIVHGNGNTYLE, a light-chain variable region CDR2: (SEQ ID NO: 37) TVSNRFS and a light-chain variable region CDR3: (SEQ ID NO: 38) FQGSHAPYT.

In a particular embodiment, the antibody or the fragment thereof comprises a heavy-chain region with SEQ ID NO: 43 and a light-chain region with SEQ ID NO: 44.

The DNA sequence of the anti-KRAS antibody from the hybridoma cell line deposited as DSM ACC3358 was obtained. The corresponding antibody is a G12D anti-KRAS monoclonal antibody. In one embodiment, the antibody or the fragment thereof comprises a heavy-chain variable region CDR1: (SEQ ID NO: 45) SYYMY, a heavy-chain variable region CDR2: (SEQ ID NO: 46) EINPSNGGTNFNEKFKS, a heavy-chain variable region CDR3: (SEQ ID NO: 47) GGYGY, a light-chain variable region CDR1: (SEQ ID NO: 29) RSSKSLLYKDGKTYLN, a light-chain variable region CDR2: (SEQ ID NO: 30) LMSTRAS and a light-chain variable region CDR3: (SEQ ID NO: 31) QQVVEYPRT.

In a particular embodiment, the antibody or the fragment thereof comprises a heavy-chain region with SEQ ID NO: 10 and a light-chain region with SEQ ID NO: 17.

In a particular embodiment, the antibody is a monoclonal antibody. More particularly, the monoclonal antibody is a humanized monoclonal antibody or a chimeric derivative for human administration.

<u>Antibody or fragment thereof</u>

The term "antibody" as used herein refers to (a) immunoglobulin polypeptides and immunologically active portions of immunoglobulin polypeptides, i.e., polypeptides of the immunoglobulin family, or fragments thereof, that contain an antigen binding site that immuno-specifically binds to a specific antigen, or (b) conservatively substituted derivatives of such immunoglobulin polypeptides or fragments that immuno-specifically bind to the antigen. Thus, both the antibody and the fragment thereof are those having antigen-binding site, i.e. are able to bind to mutated KRAS epitopes.

The term antibody also includes an "antibody derivative", which means an antibody, as defined above, that is modified by covalent attachment of a heterologous molecule such as, e.g., by attachment of a heterologous polypeptide, or by glycosylation, acetylation or phosphorylation not normally associated with the antibody, and the like.

In a particular embodiment, the antibody is a monoclonal antibody which refers to an antibody that is derived from a single cell clone, including any eukaryotic or prokaryotic cell clone, or a phage clone, and not the method by which it is produced. Thus, the term "monoclonal antibody" as used herein is not limited to antibodies produced through hybridoma technology.

The basic unit of an antibody structure is a complex of four polypeptides - two identical low molecular weight ("light") chains and two identical high molecular weight ("heavy") chains, linked together by both non-covalent associations and by disulfide bonds. Different antibodies will have anywhere from one to five of these basic units. The antibody may be represented schematically as a "Y". Each branch of the "Y" is formed by the amino terminal portion of a heavy chain and an associated light chain. The base of the "Y" is formed by the carboxy terminal portions of the two heavy chains. The node of the "Y" is referred to as the hinge region.

Five human antibody classes (IgG, IgA, IgM, IgD and IgE), and within these classes, various subclasses (e.g., IgG1, IgG2, IgG3, IgG4, IgA1 and IgA2) or subclass of immunoglobulin molecule are recognized on the basis of structural differences, such as the number of immunoglobulin units in a single antibody molecule, the disulfide bridge structure of the individual units, and differences in chain length and sequence. The class and subclass of an antibody is its isotype.

The antibody can be an intact antibody or an antigen-binding antibody fragment such as, e.g., a Fab, a F(ab'), a F(ab')2, a Fd chain, a single-chain Fv (scFv), a single-chain antibody, a disulfide-linked Fv (sdFv), a fragment comprising

either a VL or VH domain, or fragments produced by a Fab expression library. Antigen-binding antibody fragments, including single-chain antibodies, can comprise the variable region(s) alone or in combination with the entirety or a portion of the following: hinge region, CH1, CH2, CH3, CH4 and CL domains. Also, antigen-binding fragments can comprise any combination of variable region(s) with a hinge region, CH1, CH2, CH3, CH4 and CL domains. In some embodiments, an antibody fragment comprises at least one domain, or part of a domain, that includes interchain disulfide bonds.

Antibodies exist as intact immunoglobulins or as a number of well-characterized fragments produced by digestion with various peptidases. Thus, e.g., pepsin digests an antibody below (i.e. toward the Fc domain) the disulfide linkages in the hinge region to produce F(ab)'2, a dimer of Fab which itself is a light chain joined to VH-CH1 by a disulfide bond. The F(ab)'2 is reduced under mild conditions to break the disulfide linkage in the hinge region thereby converting the (Fab')2 dimer into an Fab' monomer. The Fab' monomer is essentially a Fab with part of the hinge region. While various antibody fragments are defined in terms of the digestion of an intact antibody, these and other fragments are also synthesized *de novo,* e.g., chemically by utilizing recombinant DNA methodology, by "phage display" methods, or the like. Examples of antibodies include single chain antibodies, e.g., single chain Fv (scFv) antibodies in which a variable heavy and a variable light chain are joined together (directly or through a peptide linker) to form a continuous polypeptide. Additional non-limiting examples of antibodies include nanobodies, antibody fragments, monoclonal antibodies, chimeric antibodies, reverse chimeric antibodies, etc. Antigen binding fragments include Fab, Fab', F(ab)2, dsFv, sFv, unibodies, minibodies, diabodies, tribodies, tetrabodies, nanobodies, probodies, domain bodies, unibodies, bi-specific single-chain variable fragment (bi-scFv), and the like.

"Fv" is the minimum antibody fragment that contains a complete antigen-recognition and - binding site. This region consists of a dimer of one heavy chain and one light chain variable domain in tight, non-covalent association. It is in this configuration that the three hypervariable regions of each variable domain interact to define an antigen-binding site on the surface of the VH-VLdimer. Collectively, the six hypervariable regions confer antigen-binding specificity to the antibody. However, even a single variable domain (or half of an Fv comprising only three hypervariable regions specific for an antigen) has the ability to recognize and bind antigen, although at a lower affinity than the entire binding site.

Antibody affinities may be determined as described in the examples herein below. Particular antibodies are those which bind peptide KLVVVGAVGVGK (SEQ ID NO: 40) or KLVWGADGVGK (SEQ ID NO: 41) with a Kd value of no more than about 1 x $10^{-7}$ M; particularly no more than about 1 x $10^{-8}$ M; particularly within the range between 1 and 10 nM, more particularly between 3 and 5 nM, more particularly with a Kd value of 3.9 nM and 5 nm.

Typically, the antibodies are human, rodent (e.g., mouse and rat), donkey, sheep, rabbit, goat, guinea pig, camelid, horse, or chicken. As used herein, "human" antibodies include antibodies having the amino acid sequence of a human immunoglobulin and include antibodies isolated from human immunoglobulin libraries, from human B cells, or from animals transgenic for one or more human immunoglobulin. The antibodies may be monospecific, bispecific, trispecific, or of greater multispecificity.

Particles / nanocapsules / nanoentities

A "nanoentity," as used herein, typically is a nanoentity that has an average diameter of less than 1,000 nm, e.g., less than 750 nm, less than 500 nm, less than 300 nm, less than 250 nm, less than 200 nm, less than 150 nm, or less than 100 nm. In some cases, the nanoentities have an average diameter of at least 1 nm, 5 nm, 10 nm, 50 nm, 100 nm, 500 nm, or 1,000 nm. Combinations of any of these diameters are also possible, for instance, the nanoentity has an average range of diameters of between 100 nm and 300 nm between 1,000 nm and 1 nm, between 1,000 nm and 10 nm, between 750 nm and 1 nm, between 500 nm and 10 nm, between 300 nm and 10 nm, between 250 nm and 10 nm, between 200 nm and 10 nm, between 150 nm and 10 nm, between 100 nm and 10 nm, or the like. More than one nanoentity are also present in some embodiments, and in such cases, the average (arithmetic) diameter of the plurality of nanoentities have the dimensions described here. In some cases, nanoentities having a range of diameters are present. Such nanoentities are determined by a variety of methods, such as dynamic or laser light scattering techniques. Examples of nanoentities include nanoparticles, nanocapsules, micelles, or other nanoentities such as those described herein.

In some cases, the nanoentity includes an inner core surrounded by an outer shell, e.g., exposed to the environment surrounding the nanoentity. The inner core is symmetrically or asymmetrically positioned within the nanoentity. The inner core contains, e.g., a liquid (which is, e.g., nonaqueous or aqueous), a solid and/or combinations thereof. In some embodiments, the inner core contains one or more pharmaceutical agents or drugs. For example, the inner core contains a monoclonal antibody and a small molecule such as docetaxel.

In some cases, the nanoentity is a capsule (e.g., a nanocapsule). The capsule is substantially solid or has a rubbery or gel-like shell. In addition, in some cases, the nanoentity is a particle, such as a nanoparticle. The particle is solid and has a well-defined shape. In some cases, the particle is a nanoentity having an inner core surrounded by an outer shell, e.g., the particle is a capsule. The nanocapsule has a size in the nanometer range. If the nanoparticle is generally spherical, it can also be referred to nanosphere. A nanocapsule is substantially uniform, although it has additional surface features, such as targeting moieties, penetration enhancers, antibodies, or the like, including those described herein.

In some cases, the particle is a nanoentity having an inner core surrounded by an outer shell, e.g., the particle is a capsule or a nanocapsule. In some cases, a nanocapsule has a size in the nanometer range comprising an inner core and an outer shell having a composition distinguishable from the inner core. The inner core can be, e.g., a liquid or a solid material. Often but not always, the inner core is an oil. The outer shell is formed from a continuous material and is typically not covalently attached to the inner core. In some cases, the outer shell has an average thickness of at least 1 nm, at least 2 nm, at least 3 nm, at least 5 nm, at least 10 nm, at least 20 nm, at least 30 nm, at least 50 nm, at least 100 nm, or at least 200 nm.

In a particular embodiment at least some of the plurality of nanoentities are nanocapsules with an average diameter of less than 1 micrometer.

Other nanoentities

In some cases, the nanoentity is a micelle. Typically, a micelle is formed from a plurality of surfactant or amphiphilic molecules that defines an inner portion and an exterior. For example, the surfactant molecules are arranged to have a relatively hydrophilic exterior and a relatively hydrophobic inner portion, e.g., formed from a single layer of surfactant or amphiphilic molecules. In some cases, the micelle has a size in the nanometer range. The micelle is, in some embodiments, composed by amphiphilic molecules at a concentration above the CMC (critical micellar concentration) when the micelles are dispersed in an external phase. If the external liquid phase is aqueous, the hydrophilic part of the amphiphilic molecules is oriented towards the external phase. Depending on the concentration of amphiphilic molecules, the micelles can organize themselves forming larger structures, which are clusters of micelles. Micelles are formed from surfactant molecules, e.g., having their hydrophilic portions on the surface and their hydrophobic portions pointing inwardly (or vice versa in some cases).

In some cases, the nanoentity is a liposome. A liposome can have a similar structure, but is usually formed from a double layer of surfactant or amphiphilic molecules (e.g., a lipid bilayer), and may thereby define an inner portion, a middle portion, and an outer shell; e.g., the inner portion is relatively hydrophilic, the middle portion (e.g., the outer shell of the liposome, formed by the bilayer structure of the surfactant or amphiphilic molecules) is relatively hydrophobic, and the exterior to the liposome is an aqueous or a hydrophilic environment.

If the inner portion of a nanoentity is aqueous, the aqueous liquid forming the inner portion can be made up of water containing at least one salt, in certain embodiments. Additionally, in some embodiments, the aqueous liquid forming the inner portion can contain one or more water-soluble stabilizers, preservatives, surfactants, glycols, polyols, sugars, thickening agents, gelling agents, and mixtures of these and/or other suitable excipients. These excipients are used, for instance, to improve stability of the formulation, adjust the viscosity of the final composition, control the rate of release from the inner aqueous phase, or the like.

In some embodiments, the nanoentity is a nanoemulsion. Nanoemulsions are biphasic dispersions of two immiscible liquids: either water in oil (W/O) or oil in water (O/W) droplets stabilized by at least one appropriate amphiphilic surfactant, forming droplets of nanometric sizes.

Inner core; hydrophobic compound

As used herein, the property of being "hydrophilic" is understood as the constitutional property of a molecule or functional group to penetrate into the aqueous phase or to remain therein. Accordingly, the property of being "hydrophobic" is understood as a constitutional property of a molecule or functional group to exhibit exophilic behavior with respect to water; i.e., to display the tendency to not penetrate into water, or to depart the aqueous phase. For further details reference is made to Römpp Lexikon Lacke und Druckfarben, Georg Thieme Verlag, Stuttgart, N.Y., 1998, "Hydrophilicity", "Hydrophobicity", pages 294 and 295. As used herein, the property of being "hydrophobic" can be used interchangeably with the property of being "lipophilic". However, it is known that while hydrophobic substances are usually lipophilic there are exceptions, such as in the case of silicones and fluorocarbons.

In some embodiments, the inner core comprises at least one hydrophobic compound e.g., selected from the group consisting of oil, lipophilic surfactant, fatty acid, alkane, cycloalkane, bile salt, bile salt derivatives, terpenoid, terpene, terpene-derived moieties and lipophilic vitamin.

The hydrophobic compound is particularly an oil. The oil may be volatile or non-volatile and in a particular embodiment, is selected from natural oils, semisynthetic and synthetic pharmaceutical or a combination thereof, such as animal oils, vegetable oils, hydrocarbon or silicone oils. These oils can be selected from natural, semi-synthetic and synthetic oils for pharmaceutical use, such as oils from a plant or animal origin, hydrocarbon oils or silicone oils. Oils suitable for carrying out certain embodiments of the present invention include, but are not limited to, caprylic/capric triglyceride, mineral oil, squalene oil, flavored oils, silicone oil, essential oils, water-insoluble vitamins, isopropyl stearate, butyl stearate, octyl palmitate, cetyl palmitate, tridecyl behenate, diisopropyl adipate, dioctyl sebacate, menthyl anthranilate, cetyl octanoate, octyl salicylate, isopropyl myristate, neopentyl glycol dicaprate ketols, decyl oleate, $C_{12}$-$C_{15}$ alkyl

lactates, cetyl lactate, lauryl lactate, isostearyl neopentanoate, myristyl lactate, isocetyl stearoyl stearate, octyldodecyl stearoyl stearate, hydrocarbon oils, isoparaffin, fluid paraffins, isododecane, petroleum jelly, argan oil, rapeseed oil, chili oil, coconut oil, corn oil, cottonseed oil, linseed oil, grape seed oil, mustard oil, olive oil, palm oil, fractionated palm oil, peanut oil, castor oil, pine nut oil, poppy seed oil, pumpkin seed oil, rice bran oil, safflower oil, tea tree oil, truffle oil, vegetable oil, apricot kernel oil, jojoba oil, macadamia nut oil, wheat germ oil, almond oil, soybean oil, sesame seed oil, hazelnut oil, sunflower oil, hempseed oil, rosewood oil, Kukui nut oil, avocado oil, walnut oil, fish oil, berry oil, allspice oil, juniper oil, seed oil, almond seed oil, anise seed oil, celery seed oil, cumin seed oil, nutmeg seed oil, basil leaf oil, bay leaf oil, cinnamon leaf oil, common sage leaf oil, eucalyptus leaf oil, lemon leaf oil, melaleuca leaf oil, oregano oil, patchouli leaf oil, peppermint leaf oil, pine needle oil, rosemary leaf oil, spearmint oil, tea tree leaf oil, thyme oil, flower oil, chamomile oil, clary sage oil, clove oil, geranium flower oil, hyssop flower oil, jasmine oil, lavender oil, mauka flower oil, marjoram flower oil, orange flower oil, rose flower oil, ylang-ylang flower oil, bark oil, cassia bark oil, cinnamon bark oil, sassafras bark oil, wood oil, camphor wood oil, cedarwood oil, rosewood oil, sandalwood oil, ginger wood oil, tall oil, castor oil, myrrh oil, peel oil, Bergamot peel oil, grapefruit peel oil, lemon peel oil, lime peel oil, orange peel oil, tangerine peel oil, root oil, valerian oil, oleic acid, linoleic acid, oleyl alcohol, isostearyl alcohol, ethyl oleate, medium-chain triglycerides such as mixtures of decanoyl- and octanoyl glycerides (Miglyol® 810N, Miglyol® 812N, Kollisolv® MCT, Captex® 300, Captex® 355, Labrafac® Lipophile WL1349), Labrafil® M 2125 CS (Linoleoyl macrogol-6 glycerides), Labrafil® M2130 CS (Lauroyl macrogol-6 glycerides), Labrafil® M 1944 CS (oleoyl polyoxyl-6 glycerides), Labrafac® PG (propylene glycol dicaprylocaprate), Rylo® (mixture of fatty acids), Peceol® (glycerol monooleate) and Maisine® (glycerol monolinoleate), synthetic or semi-synthetic derivatives thereof and combinations thereof. In some cases, the oil is one or more of peanut oil, cottonseed oil, olive oil, castor oil, soybean oil, safflower oil, sesame oil, corn oil, palm oil, alpha-tocopherol (vitamin E), isopropyl myristate, squalene, Miglyol®, Labrafil®, Labrafac®, Peceol®, Captex®, Kollisolv® MCT and Maisine® or mixtures thereof. Other suitable oils include oils from the terpene family formed by isoprene units (2-methylbuta-1,3-diene) and sub-divided according to their carbon atoms: hemiterpenes (C5), monoterpenes (C10), sesquiterpenes (C15), diterpenes (C20), sesterterpenes (C25), triterpenes (C30), tetraterpenes (C40, carotenoids) and polyterpenes, vitamin A, squalene, etc. In some embodiments, the nonaqueous liquid forming the inner portion can contain water-insoluble stabilizers, preservatives, surfactants, organic solvents and mixtures thereof to provide maximum stability of the formulation. Combinations of one or more of these and/or other oils are also possible in various embodiments.

In one embodiment, the inner core comprises an oil, and particularly a caprylic/capric triglyceride (such as Miglyol® 812N).

Outer shell; polymer

The advantage of nanocapsule systems with respect to emulsion systems is the presence of a polymer coating the oily cores that may confer greater stability and protection against aggregation, a change in the drug release profile of the drug associated, an increased cellular internalization and specific interaction with certain cell types. A variety of polymers can be used in accordance to certain embodiments of the invention.

In some embodiments the polymer is selected from the group consisting of: polysialic acid (PSA), hyaluronic acid (HA), polyglutamic acid (PGA) and/or pegylated-polyglutamic acid (PGA-PEG), polylactic acid (PLA) and/or pegylated polylactic acid (PLA-PEG), poly(aspartic acid) (PASP) and/or pegylated- poly(aspartic acid) (PASP-PEG), poly(lactic-co-glycolic acid) (PLGA) and/or pegylated poly(lactic-co-glycolic acid) (PLA-PEG), alginic acid (ALG) and/or pegylated alginic acid (ALG-PEG), polymalic acid (PLMA) and/or pegylated polymalic acid (PLMA-PEG), and mixtures thereof. Combinations of these and/or other polymers are also used in certain embodiments.

In particular embodiments, the polymer is selected from the group consisting of: polysialic acid, hyaluronic acid, polyglutamic acid, polyaspartic acid, polymalic acid, alginic acid, polylactic acid, polylactic-co-glycolic acid, pegylated forms thereof, and mixtures thereof. In particular embodiments, the polymer is PSA or HA.

The polymer is evenly distributed throughout the entity, or concentrated within certain regions of the entity, e.g., in the outer shell of a capsule, or other outer surface of an entity. In some cases, at least 50 wt% of a portion of an entity, such as a shell, comprises the polymer, and in certain cases, at least 60 wt%, at least 70 wt%, at least 75 wt%, at least 80 wt%, at least 85 wt%, at least 90 wt%, at least 95 wt%, or at least 99 wt% of the portion of the entity can comprise the polymer.

Polymer - PSA

In a particular embodiment, the polymer is PSA. PSA is generally composed of a plurality of sialic acid units, often bonded together to form a polymer via 2-->8 and/or 2-->9 bonding, although other bonding arrangements are also possible. Typically, there are at least 2, at least 4, at least 6, at least 8, at least 10, at least 15, at least 20, at least 25, at least 30, at least 40, at least 50, at least 75, at least 100, at least 200, at least 300, at least 400, or at least 500 sialic acid units bonded together to form PSA. In some cases, the PSA has no more than 1000, no more than 500, no more than 200, no more than 100, no more than 50, no more than 30, or no more than 10 sialic acid units bonded together to form the PSA.

Combinations of any of these are also possible, e.g., a PSA has between 2 and 100 sialic acid units that are bonded together. It should be noted that the sialic acid units need not be identical, and can independently be the same or different, even within the same PSA molecule. It should also be noted that a PSA need not necessarily be a straight (linear) chain, and various branching arrangements are also possible. For instance, a sialic acid unit is bonded to 3 or more different sialic acid units, thereby creating a branch point within the PSA molecule.

As non-limiting examples, the PSA has different molecular weights, e.g., 4 kDa, 30 kDa, 95 kDa, etc. In some cases, the PSA contains more than 300 sialic acid units. As additional non-limiting examples, the PSA has a molecular weight of at least 1 kDa, at least 3 kDa, at least 5 kDa, at least 10 kDa, at least 20 kDa, at least 25 kDa, at least 30 kDa, at least 40 kDa, at least 50 kDa, at least 60 kDa, at least 70 kDa, at least 75 kDa, at least 80 kDa, at least 90 kDa, at least 100 kDa etc. In some cases, the PSA has a molecular weight of no more than 100 kDa, no more than 90 kDa, no more than 80 kDa, no more than 75 kDa, no more than 70 kDa, no more than 60 kDa, no more than 50 kDa, no more than 40 kDa, no more than 30 kDa, no more than 25 kDa, no more than 20 kDa, no more than 10 kDa, no more than 5 kDa, no more than 3 kDa, or no more than 1kDa. Combinations of any these are also possible, e.g., the PSA has a molecular weight between about 1 kDa and about 100 kDa, between about 5 kDa and about 80 kDa, or between about 10 kDa and about 50 kDa, etc. (Unless indicated to the contrary, molecular weights described herein are number average molecular weights).

It should also be noted that polysialic acids do not need always to be identical. For example, in some embodiments, the PSAs have different numbers of sialic acid units, and/or there are different sialic acid units in different PSA molecules that are present. In some cases, one or a few types of PSA molecules may be present, e.g., one or more forms comprise at least 30%, at least 40%, at least 50%, at least 60%, at least 70%, at least 80%, at least 90%, or more of the PSA molecules that are present, i.e., on a molar basis.

Non-limiting examples of sialic acid units that are present within a PSA include, but are not limited to, N-acetylneuraminic acid (Neu), 2-keto-3-deoxynonic acid (Kdn), lactaminic acid, N-sialic acid, and/or O-sialic acid. Other examples include N-glycolylneuraminic (Neu5Gc), 9-O-acetyl-8-O-methyl-N-acetylneuraminic acid (Neu5,9Ac28Me), and 7,8,9-tri-O-acetyl-N-glycolylneuraminic acid (Neu5Gc7,8,9Ac3). "Sia" generally denotes an unspecified sialic acid unit. In some embodiments, the sialic acid units include any derivative of neuraminic acid (a 9-carbon sugar), including the 43 derivatives typically found in nature. These include, but are not limited to, Neu; Neu5Ac; Neu4,5$Ac_2$; Neu5,7$Ac_2$; Neu5,8$Ac_2$; Neu5,9$Ac_2$; Neu4,5,9$Ac_3$; Neu5,7,9$Ac_3$; Neu5,8,9$Ac_3$; Neu5,7,8,9$Ac_4$; Neu5Ac9Lt; Neu4,5$Ac_2$9Lt; Neu5Ac8Me; Neu5,9$Ac_2$8Me; Neu5Ac8S; Neu5Ac9P; Neu2en5Ac; Neu2en5,9$Ac_2$; Neu2en5Ac9Lt; Neu2,7an5Ac; Neu5Gc; Neu4Ac5Gc; Neu7Ac5Gc; Neu8Ac5Gc; Neu9Ac5Gc; Neu7,9$Ac_2$5Gc; Neu8,9$Ac_2$5Gc; Neu7,8,9$Ac_3$5Gc; Neu5Gc9Lt; Neu5Gc8Me; Neu9Ac5Gc8Me; Neu7,9$Ac_2$5Gc8Me; Neu5Gc8S; Neu5GcAc; Neu5GcMe; Neu2en5Gc; Neu2en9Ac5Gc; Neu2en5Gc9Lt; Neu2en5Gc8Me; Neu2,7an5Gc; Neu2,7an5Gc8Me; Kdn; and Knd9Ac. In one set of embodiments, each of the sialic acid units (prior to polymerization to form PSA) can independently have the following structure:

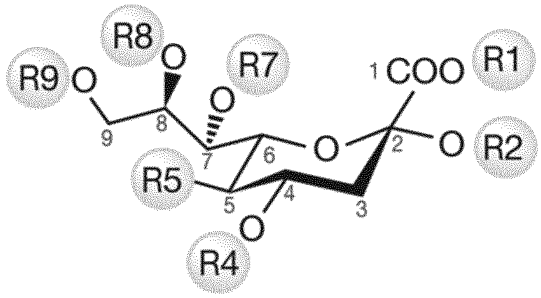

$R^1$ is H; an alpha linkage to Gal(3/4/6), GalNAc(6) (N-acetylgalactosamine), GlcNAc(4/6), Sia (8/9), or 5-O-Neu5Gc; an oxygen linked to C-7 in 2,7-anhydro molecule; or an anomeric hydroxyl eliminated in Neu2en5Ac (double bond to C-3). $R^2$ is H; an alpha linkage to Gal(3/4/6), GalNAc(6), GlcNAc(4/6), Sia (8/9), or 5-O-Neu5Gc; an oxygen linked to C-7 in 2,7-anhydro molecule; or an anomeric hydroxyl eliminated in Neu2en5Ac (double bond to C-3). $R^4$ is H; -acetyl; an anhydro to C-8; Fuc (fucose); or Gal (galactose). $R^5$ is an amino; N-acetyl; N-glycolyl; hydroxyl; N-acetimidoyl; N-glycolyl-O-acetyl; N-glycolyl-O-methyl; or N-glycolyl-O-2-Neu5Gc. $R^7$ is H; -acetyl; an anhydro to C-2; or substituted by amino and N-acetyl in Leg (legionaminic acid). $R^8$ is H; -acetyl; an anhydro to C-4; -methyl; -sulfate; Sia (sialic acid); or Glc (glucose). $R^9$ is H; -acetyl; -lactyl; -phosphate; -sulfate; Sia; or OH substituted by H in Leg. In some cases, the PSA is colominic acid (where only 2-->8 bonding is present).

As used herein, sialic acid includes, but is not limited to, water-soluble salts and water-soluble derivatives of sialic acid. For example, the sialic acid salt is the sodium salt, the potassium salt, the magnesium salt, the calcium salt, or the zinc salt. In one embodiment, at least some of the sialic acid is present as a sodium salt. Combinations of multiple types of sialic acids are also used, e.g., as subunits of a PSA, and/or as different molecules of PSA. In one set of embodiments, at least

some of the sialic acid within PSA is modified (however, it should be understood that in other embodiments, the PSA is not necessarily modified). For instance, in some cases, one or more sialic acid units are modified, e.g., by attachment to polyethylene glycol, alkyl or other hydrophobic moieties, or the like. Hydrophobic moieties include hydrophobic molecules or portions thereof, e.g., an alkyl group, such as those discussed herein.

Polymer - Hyaluronic acid, HA

In one set of embodiments, the polymer comprises hyaluronic acid. Hyaluronic acid is a linear polymer comprising the repetition of a disaccharide structure formed by the alternating addition of D-glucuronic acid and D-N-acetylglucosamine bound by alternating beta-1,4 and beta-1,3 glycosidic bonds as shown in the following formula:

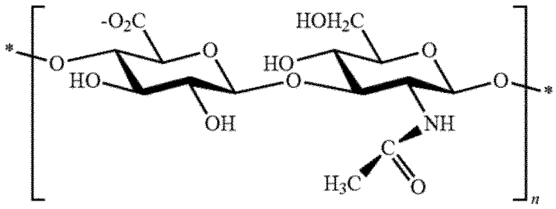

wherein the integer n represents the degree of polymerization, i.e., the number of disaccharide units in the hyaluronic acid chain. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. Combinations of any of these are also possible, e.g., n is between 2 and 100. It should be noted that the hyaluronic acid units need not be identical, and can independently be the same or different, even within the same hyaluronic acid chain. It should also be noted that hyaluronic acid need not necessarily be a straight (linear) chain, and various branching arrangements are also possible.

Thus, hyaluronic acid with a wide range of molecular weights can be used. As non-limiting examples, the hyaluronic acid has different molecular weights, e.g., 4 kDa, 30 kDa, 95 kDa, etc. For example, hyaluronic acid has a molecular weight of at least 1 kDa, at least 3 kDa, at least 5 kDa, at least 10 kDa, at least 20 kDa, at least 25 kDa, at least 30 kDa, at least 40 kDa, at least 50 kDa, at least 60 kDa, at least 70 kDa, at least 75 kDa, at least 80 kDa, at least 90 kDa, at least 100 kDa etc. In some cases, hyaluronic acid has a molecular weight of no more than 100 kDa, no more than 90 kDa, no more than 80 kDa, no more than 75 kDa, no more than 70 kDa, no more than 60 kDa, no more than 50 kDa, no more than 40 kDa, no more than 30 kDa, no more than 25 kDa, no more than 20 kDa, no more than 10 kDa, no more than 5 kDa, no more than 3 kDa, or no more than 1kDa. Combinations of any these are also possible, e.g., the hyaluronic acid has a molecular weight between about 1 kDa and about 100 kDa, between about 5 kDa and about 80 kDa, or between about 10 kDa and about 50 kDa, etc.

Hyaluronic acid, as used herein, also includes its conjugated base (hyaluronate). This conjugated base can be an alkaline salt of hyaluronic acid including inorganic salts such as, e.g., sodium salt, potassium salt, calcium salt, ammonium salt, magnesium salt, aluminium salt and lithium salt, organic salts such as basic amino acid salts at neutral pH. In some cases, the salts are pharmaceutically acceptable. In one embodiment, the alkaline salt is the sodium salt of hyaluronic acid. Combinations of multiple types of hyaluronic acid are also used, e.g., as subunits of a hyaluronic acid chain, and/or as different molecules of hyaluronic acid.

Thus, the hyaluronic acids need not always be identical. For example, in some embodiments, the hyaluronic acids have different numbers of hyaluronic acid units (such as those described above), and/or there are different hyaluronic acid units in different hyaluronic acid chains that are present. In some cases, one or more types of hyaluronic acid molecules are present, e.g., one or more forms comprise at least 30%, at least 40%, at least 50%, at least 60%, at least 70%, at least 80%, at least 90%, or more of the hyaluronic acid molecules that are present, i.e., on a molar basis.

In some embodiments, at least some of the hyaluronic acid units are modified (however, it should be understood that in other embodiments, the hyaluronic acid is not necessarily modified). For instance, in some cases, one or more hyaluronic acid units are modified, e.g., by attachment to polyethylene glycol, alkyl or other hydrophobic moieties, or the like. Hydrophobic moieties include hydrophobic molecules or portions thereof, e.g., an alkyl group, such as those discussed herein.

Polymer - Polyglutamic acid, PGA

In one set of embodiments, the polymer comprises polyglutamic acid (PGA). PGA is a polymer of the amino acid glutamic acid (GA). Apart from molecular weight (Mw), the ratio of D- to L-glutamic acid monomers and molecular structure

are important chemical characteristics of PGA. PGA exists as two different structures, as depicted below:

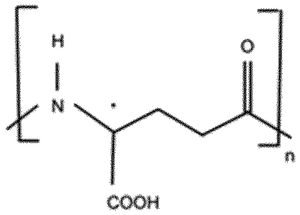

Poly-γ-Glutamic Acid

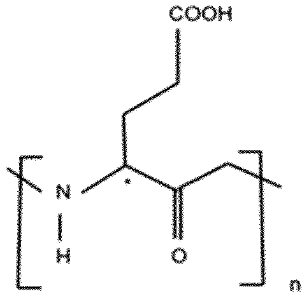

Poly-α-Glutamic acid

wherein the integer n represents the degree of polymerization, i.e., the number of glutamic acid monomers in the PGA chain. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. Thus, polyglutamic acids with a wide range of molecular weights can be used. As non-limiting examples, the polyglutamic acid has different molecular weights, for example, the polyglutamic acid has a molecular weight of at least 1 kDa, at least 3 kDa, at least 5 kDa, at least 10 kDa, at least 20 kDa, at least 25 kDa, at least 50 kDa, at least 60 kDa, at least 70 kDa, at least 80 kDa, at least 90 kDa, at least 100 kDa, etc. Combinations of any of these are also possible. It should also be noted that glutamic units need not necessarily be a straight (linear) chain, and various branching arrangements are also possible.

Poly-α-glutamic acid (α-PGA) can be chemically synthesized, while γ-PGA can only be obtained via microbial production.

As used herein, polyglutamic acid includes, but is not limited to, water-soluble salts and water-soluble derivatives of polyglutamic acid (e.g. sodium salt, potassium salt, magnesium salt, calcium salt). In one set of embodiments, at least some of the glutamic acids within PGA are modified (however, it should be understood that in other embodiments, the PGA is not necessarily modified). For instance, in some cases, one or more glutamic acid units are modified, e.g., by attachment to polyethylene glycol, alkyl or other hydrophobic moieties, or the like.

Polymer - Polyaspartic acid, PASP

In one set of embodiments, the polymer comprises polyaspartic acid (PASP). PASP is a poly(amino acid) with a protein-like amide bond in its backbone, and a carboxylic acid as a pendant group in each repeating unit. In nature, PASP has been found in as fragments of larger proteins with length up to 50 amino acids. PASP can be synthesized by different methods. The polymer is present in different forms such as α,β and L, D isomers.

L, α

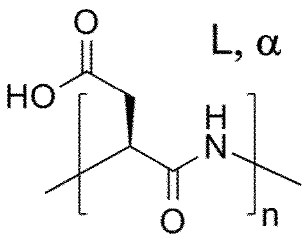

L, β

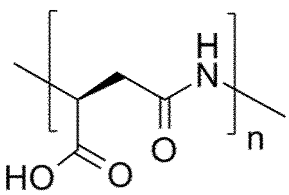

D, α

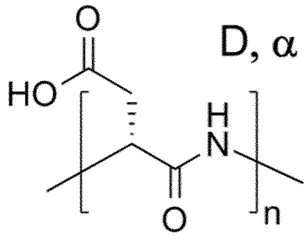

D, β

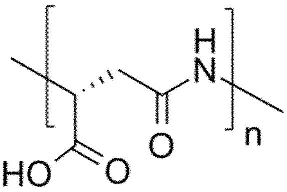

wherein the integer n represents the degree of polymerization, i.e., the number of aspartic acid monomers in the PASP chain. Any number of aspartic acid units are present within the polymer. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. As non-limiting examples, the polyglutamic acid has different molecular weights, for example, the polyglutamic acid has a molecular weight of at least 1 kDa, at least 3 kDa, at least 5 kDa, at least 10 kDa, at least 20 kDa, at least 25 kDa, at least 50 kDa, at least 60 kDa, at least 70 kDa, at least 80 kDa, at least 90 kDa, at least 100 kDa, etc. Combinations of any of these are also possible. It should also be noted that other amino acids maybe present within the PASP chain, and the polymer is straight or branched.

As used herein, polyaspartic acid includes, but is not limited to, water-soluble salts and water-soluble derivatives of polyaspartic acid (e.g. sodium salt, potassium salt, magnesium salt, calcium salt). In one set of embodiments, at least some of the aspartic acids within PASP are modified (however, it should be understood that in other embodiments, the PASP is not necessarily modified). For instance, in some cases, one or more aspartic acid units are modified, e.g., by attachment to polyethylene glycol, alkyl or other hydrophobic moieties, or the like.

Polymer - Polymalic acid, PMLA

In one set of embodiments, the polymer comprises polymalic acid (PMLA). PLMA is a carboxylic-functionalized polyester that can be produced by either chemical synthesis or biological fermentation from the slime mold *Physarum polycephalum.* PMLA is a completely biodegradable polymer that is metabolized to water and carbon dioxide in the citric acid cycle. Both α- and β-structures, either racemic or optically pure, may be obtained by chemical methods, whereas microorganisms exclusively generate PMLA of extremely high optical purity. Pendant carboxylic acid groups can be chemically modified to introduce molecules of interest. Structure of the different forms of PLMA is depicted below:

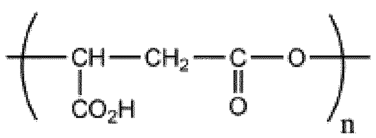

Poly(β-malic acid)

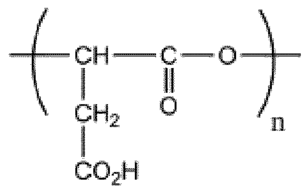

Poly(α-malic acid)

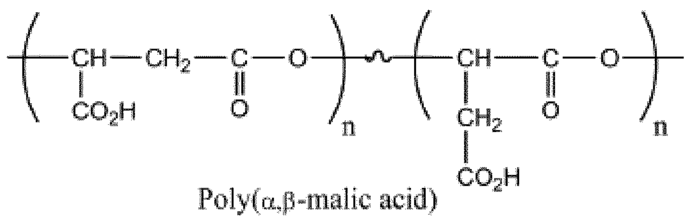

Poly(α,β-malic acid)

wherein the integer n represents the degree of polymerization, i.e., the number of malic acid units in the PLMA chain. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. Thus, polymalic acids with a wide range of molecular weights can be used. As non-limiting examples, the polymalic acid has different molecular weights, for example, the polymalic acid has a molecular weight of at least 1 kDa, at least 3 kDa, at least 5 kDa, at least 10 kDa, at least 20 kDa, at least 25 kDa, at least 50 kDa, at least 60 kDa, at least 70 kDa, at least 80 kDa, at

least 90 kDa, at least 100 kDa, etc. Combinations of any of these are also possible. It should also be noted that malic acid units need not necessarily be a straight (linear) chain, and various branching arrangements are also possible.

As used herein, polymalic acid includes, but is not limited to, water-soluble salts and water-soluble derivatives of polymalic acid (e.g. sodium salt, potassium salt, magnesium salt, calcium salt). In one set of embodiments, at least some of the malic acids within PLMA are modified (however, it should be understood that in other embodiments, the PLMA is not necessarily modified). For instance, in some cases, one or more malic acid units are modified, e.g., by attachment to polyethylene glycol, alkyl or other hydrophobic moieties, or the like.

Polymer - Alginic acid, ALG

In one set of embodiments, the polymer comprises alginic acid (ALG). ALG is a hydrophilic polysaccharide distributed widely in the cell walls of brown algae. Its salts are known as alginates. Alginates are actually block copolymers, and that the ratio of guluronate to mannuronate varies depending on the natural source. Alginate is known to be a whole family of linear copolymers containing blocks of (1,4)-linked β-D-mannuronate (M) and α-L-guluronate (G) residues. The blocks are composed of consecutive G residues (GGGGGG), consecutive M residues (MMMMMM), and alternating M and G residues (GMGMGM), as depicted below (Lee and Mooney, 2012).

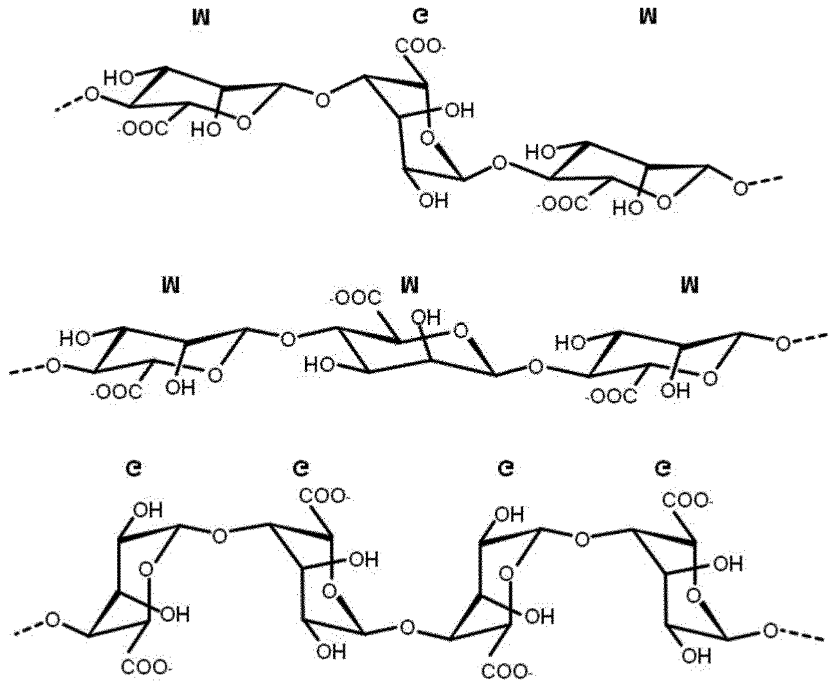

As non-limiting examples, alginic acid has different molecular weights, for example, the alginate has a molecular weight of at least 1 kDa, at least 3 kDa, at least 5 kDa, at least 10 kDa, at least 20 kDa, at least 25 kDa, at least 50 kDa, at least 60 kDa, at least 70 kDa, at least 80 kDa, at least 90 kDa, at least 100 kDa, etc. Combinations of any of these are also possible.

As used herein, alginic acid includes, but is not limited to, water-soluble salts and water-soluble derivatives of alginic acid (e.g. sodium salt, potassium salt, magnesium salt, calcium salt). In one set of embodiments, at least some of the guluronate and/or mannuronate residues within ALG are modified (however, it should be understood that in other embodiments, the ALG is not necessarily modified). For instance, in some cases, one or more guluronate and/or mannuronate residues are modified, e.g., by attachment to polyethylene glycol, alkyl or other hydrophobic moieties, or the like.

Polymer - Polylactic acid, PLA

In one set of embodiments, the polymer comprises the polylactic acid (PLA) polyester. Structure of PLA is depicted below:

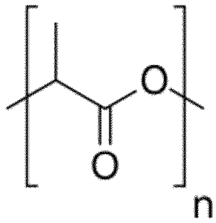

wherein the integer n represents the degree of polymerization, i.e., the number of lactic/ lactide acid units in the PLA chain. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. Thus, PLA with a wide range of molecular weights can be used. As non-limiting examples, the PLA has different molecular weights, for example, the PLA has a molecular weight of at least 1 kDa, at least 3 kDa, at least 5 kDa, at least 10 kDa, at least 20 kDa, at least 25 kDa, at least 50 kDa, at least 60 kDa, at least 70 kDa, at least 80 kDa, at least 90 kDa, at least 100 kDa, etc. Combinations of any of these are also possible. It should also be noted that lactic/ lactide acid units need not necessarily be a straight (linear) chain, and various branching arrangements are also possible.

Polymer - Polylactic-co-glycolic, PLGA

In one set of embodiments, the polymer comprises the polylactic-co-glycolic (PLGA) polyester. Structure of PLGA is depicted below:

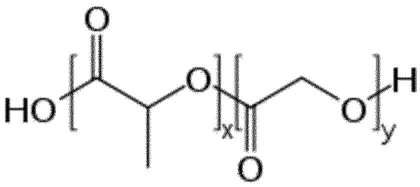

wherein the integer x represents the number of units of lactic acid and the integer y represents the number of units of glycolic acid. Depending on the ratio of lactic to glycolic used for the polymerization, different forms of PLGA can be obtained, and these are usually identified in regard to the molar ratio of the monomers used. As non-limiting examples, PLGA is 75:25 (copolymer whose composition is 75% lactic acid and 25% glycolic acid), 50:50 (copolymer whose composition is 50% lactic acid and 50% glycolic acid), and 25:75 (copolymer whose composition is 25% lactic acid and 75% glycolic acid).

In some cases, one or more lactic acid and/or glycolic acid monomers in PLA or PLGA are modified, e.g., by attachment to polyethylene glycol, alkyl or other hydrophobic moieties, or the like.

Pegylated polymers, Polymers-PEG

In one set of embodiments, the polymer comprises poly(ethylene glycol) (PEG). Thus, in some cases, the PEG is conjugated to, for example, HA, PGA, PASP, PLMA, ALG, PLA or PLGA, e.g., to form HA-PEG, PGA-PEG, PASP-PEG, PLMA-PEG, ALG-PEG, PLA-PEG or PLGA-PEG. However, in other cases, PEG is present, i.e., not conjugated to the polymers. PEG, in its most common form, is a polymer having a formula:

$$H\text{-}(O\text{-}CH_2\text{-}CH_2)_n\text{-}OH,$$

where n is an integer representing the PEG polymerization degree. For the formation of the conjugate polymer-PEG, one or two of the two terminal hydroxyl groups are modified. The modified PEGs, e.g., as follows:

$$X^1\text{-}(O\text{-}CH_2\text{-}CH_2)_n\text{-}X^2,$$

where $X^1$ is hydrogen or a hydroxyl protecting group blocking the OH radical function for subsequent reactions. For example, n is at least 2, at least 4, at least 6, at least 8, at least 10, at least 15, at least 20, at least 25, at least 30, at least 40, at least 50, at least 75, at least 100, at least 200, at least 300, at least 400, or at least 500. In some cases, n is no more than 1000, no more than 500, no more than 200, no more than 100, no more than 50, no more than 30, or no more than 10. Combinations of any of these are also possible, e.g., n is between 2 and 100. The protecting groups of hydroxyl radicals are

widely known in the art.

Pegylation of the polymers can be performed using any suitable method available in the art. Such polymers are available in a variety of molecular weights. For example, a suitable molecular weight for PEG or Polymer-PEG is between about 1 kDa and about 100 kDa, between about 5 kDa and about 80 kDa, between about 10 kDa and about 50 kDa. As additional non-limiting examples, the PEG or Polymer-PEG has a molecular weight of at least 1 kDa, at least 5 kDa, at least 10 kDa, at least 20 kDa, at least 30 kDa, at least 40 kDa, at least 50 kDa, at least 60 kDa, at least 70 kDa, at least 80 kDa, at least 90 kDa, at least 100 kDa etc. Combinations of any of these are also possible.

In some embodiments, the proportion of PEG in the polymer (e.g. HA, PGA, PASP, PLMA, ALG, PLA, PLGA, etc) can be between about 10% and 90% (w/w) relative to the total weight of the polymer, between about 15% and 80%, between about 20% and 70%, or about 20%, about 22%, about 24%, about 26%, about 28%, about 30%, about 32%, about 34%, about 36%, about 38%, about 40%, about 42%, about 44%, about 46%, about 48%, about 50%, about 52%, about 54%, about 56%, about 58%, or about 60%.

Targeting moiety

In one embodiment, the entity also comprises a targeting moiety, although it should be noted that in some embodiments, no targeting moiety is present. The targeting moiety (if present) is used to target delivery of entities, e.g., to certain cell populations within a subject. For instance, the targeting moiety facilitates the access of the nanoentities to one or more types of cells, e.g., a cancer cell, an endothelial cell, and/or an immune cell. In some embodiments, the targeting moiety allows targeting of the entity to a specific location within the subject, for example, a specific organ or a specific cell type (e.g., to a tumor or cancer cells). In some cases, the entities are internalized by the cells with no need for targeting moieties, and in some other cases, internalization is facilitated by the targeting moiety. In some embodiments, more than one type of targeting moiety is present. In some embodiments, a targeting moiety includes a cell- and/or tumor/tissue-penetrating peptide. However, it should be understood that in certain embodiments, the targeting moiety may not necessarily also facilitate internalization.

A wide variety of targeting moieties may be used in various embodiments. For example, the targeting moieties include peptides, proteins, aptamers, antibodies (including monoclonal antibodies, nanobodies and antibody fragments), nucleic acids, organic molecules, ligands, or the like. Some targeting moieties may be seen, e.g., in Bertrand, et al., 2014, et al., 2016, and Zhou et al., 2016.

In one set of embodiments, for example, the targeting moiety is a peptide, e.g., having a length of no more than 50 amino acids, no more than 40 amino acids, no more than 30 amino acids, no more than 20 aminoacids or no more than 10 amino acids.

In one set of embodiments, the targeting moiety is a cell-penetrating and/or a tumor/tissue-penetrating peptide. A cell-penetrating peptide has the capacity to penetrate a cell membrane. A tumor-penetrating peptide has the capacity of favouring the deep penetration of drug payloads into the tumor. In some cases, the cell-penetrating and/or tumor/tissue-penetrating peptide also facilitates the targeting of the nanoentities to the cells.

The "natural amino acids," as used herein, are the 20 amino acids commonly found in nature, typically in the L-isomer, i.e., alanine ("Ala" or "A"), arginine ("Arg" or "R"), asparagine ("Asn" or "N"), aspartic acid ("Asp" or "D"), cysteine ("Cys" or "C"), glutamine ("Gln" or "Q"), glutamic acid ("Glu" or "E"), glycine ("Gly" or "G"), histidine ("His" or "H"), isoleucine ("Ile" or "I"), leucine ("Leu" or "L"), lysine ("Lys" or "K"), methionine ("Met" or "M"), phenylalaine ("Phe" or "F"), proline ("Pro" or "P"), serine ("Ser" or "S"), threonine ("Thr" or "T"), tryptophan ("Trp" or "W"), tyrosine ("Tyr" or "Y"), and valine ("Val" or "V").

Some tumor/tissue-penetrating peptides may be found, for example, in Ruoslahti 2017. For example, the peptides can include a C-terminal "C-end Rule" (CendR) sequence motif (R/K)XX(R/K) which is associated with the capacity to enhance the permeability of tumor blood vessels and tumor tissues *via* binding to receptors, such as for example neuropilin-1 (NRP-1). Each X in this sequence is independently an amino acid or no amino acid.

In some cases, the targeting moiety comprises a sequence $Z^1X^1X^2Z^2$, where $Z^1$ is R or K, $Z^2$ is R or K, and $X^1$ and $X^2$ are each independently an amino acid residue or no amino acid residue. In some cases, one or both ends of the peptide comprise other amino acids, e.g., as in the structures $J^1Z^1X^1X^2Z^2$, $Z^1X^1X^2Z^2J^2$, or $J^1Z^1X^1X^2Z^2J^2$, wherein each of $J^1$ and $J^2$ is independently an amino acid sequence (e.g., comprising 1, 2, 3, 4, 5, 6, or more amino acid residues) or an or an aliphatic carbon chain. The aliphatic carbon chain contains carbon and hydrogen atoms in any suitable sequence, e.g., straight-chained or branched, and is saturated or unsaturated. For instance, in one set of embodiments, the aliphatic carbon chain is a straight alkyl chain having a formula e.g., $-(CH_2)_n-$, n being 1, 2, 3, 4, 5, 6, 7, 8, 9, 10, 11, 12, or another positive integer. In addition, in some cases, the sequence ends with a cysteine residue, e.g., as in $CJ^1Z^1X^1X^2Z^2$, $CZ^1X^1X^2Z^2J^2$, or $CJ^1Z^1X^1X^2Z^2J^2$.

Non-limiting examples of CendR peptides include but are not limited to Lyp-1, cLyp1, tLyp-1, iNGR, iRGD, RPARPAR, TT1, linear TT1, F3, or CRGRRST. Optionally, other amino acids are present in the peptide as well. Lyp-1 has a sequence CGNKRTRGC (SEQ ID NO: 1). In some embodiments, the two Cys residues are bonded to each other via a

disulfide bridge, thereby forming a circular structure, thus being also known as cLyp-1. In some cases, only a portion of the Lyp-1 sequence is present, e.g., as in the case of truncated Lyp-1 or tLyp-1 (CGNKRTR) (SEQ ID NO: 2). iNGR has a sequence CRNGRGPDC (SEQ ID NO: 4), where the two cysteines are linked together. iRGD has a sequence (CRGDKGPDC) (SEQ ID NO: 5), where the two cysteines are linked together, and iRGD2 has a sequence CRGDRGPDC (SEQ ID NO: 6). RPARPAR has a sequence RPARPAR (SEQ ID NO: 7). TT1 has a sequence CKRGARSTC (SEQ ID NO: 8), where the two cysteines are linked together. Linear TT1 has a sequence AKRGARSTA (SEQ ID NO: 9). F3 has a sequence KDEPQRRSARLSAKPAPPKPEPKPKKAPAKK (SEQ ID NO: 32). CRGRRST has a sequence CRGRRST (SEQ ID NO: 3)

In some embodiments, the targeting moiety comprises the integrin-binding RGD sequence motif, which binds to integrins expressed in tumor endothelial cells. Optionally, other amino acids may be present in the peptide as well. Both linear and cyclic RGD peptides can be used as targeting moieties. Cyclization of RGD peptides may be performed, for example, via linkers such as S-S disulfide, thioether, and rigid aromatic rings. Some RGD peptides and derivatives may be found, e.g., in Kapp, et al., 2017. Non limiting examples of RGD peptides include but are not limited to RGD, RGDS (SEQ ID NO: 14), GRGD (SEQ ID NO: 15), GRGDS (SEQ ID NO: 16), GRGDSP (SEQ ID NO: 13), GRGDSPK (SEQ ID NO: 26), GRGDNP (SEQ ID NO: 27, GRGDTP (SEQ ID NO: 28), RGD-4C (CDCRGDCFC, SEQ ID NO: 11), RGD-10 (GARYCRGDCFDGR, SEQ ID NO: 12), cyclic RGD pentapeptides, cyclic hexapeptides, Cilengitide (c(RGDf(NMe)V)) or the aforementioned CendR peptide iRGD. Cyclic RGD pentapeptides may be characterized by the formula c(RGDxX), where the x residue is an aromatic aminoacid in the D-configuration (essential for $\alpha v\beta 3$-integrin binding affinity), such as for example the sequences c(RGDfV), c(RGDfK), c(RGDyK) or c(RGDfC).

Peptides including any of the sequences disclosed above exhibit, in some embodiments, cell- or tissue-penetrating activity, and particularly in tumor tissue. One set of embodiments is generally directed to the association of cell-penetrating peptides with no targeting properties, e.g., to provide at least some of the nanoentities with cell- or tissue-penetrating activity when non-systemically administered to a subject (e.g. intra-tumoral, nasal, topical, intra-peritoneal, vaginal, rectal, oral, pulmonary, ocular, etc.), or when administered *in vitro* or ex vivo, e.g., to living cells or tissues. In some cases, some of the nanocapsules polymer (e.g., PSA, HA, PGA) is linked to cell-penetrating peptides, e.g. by covalent or non-covalent association.

Some cell-penetrating peptides may be found, e.g., in Zhang et al., 2016, and Regberg et al., 2012.

Cell-penetrating peptides useful for certain embodiments of the present invention are selected from, although they are not limited to, TAT, mTAT (C-5H-TAT-5H-C), G3R6TAT, TAT(49-57), TAT(48-60), MPS, VP22, Antp, gH625, arginine-rich CPPs (e.g. octarginine, polyarginine, stearyl-polyarginine, HIV-1 Rev34-50, FHV coat35-49) penetratin, penetratin-Arg, penetratin-Lys, SR9, HR9, PR9, H(7)K(R(2)), Pep-1, Pep-3, transportan, transportan10, pepFect, pVEC, JB577, TD-1, MPG8, CADY, YTA2, YTA4, SynB1, SynB3, PTD-4, GALA, SPACE, or the like.

Cell-penetrating peptides can also be coupled with targeting moieties. Some non-limiting examples are PEGA (CPGPEGAGC) (SEQ ID NO: 18), CREKA (SEQ ID NO: 19), RVG (YTIWMPENPRPGTPCDIFTNSRGKRASNG) (SEQ ID NO: 20), DV3 (LGASWHRPDKG) (SEQ ID NO: 21), DEVDG (SEQ ID NO: 22), ACPP-MMP-2/9 (PLGLAG) (SEQ ID NO: 23), ACPP-MMP-2 (IAGEDGDEFG) (SEQ ID NO: 24), R8-GRGD (SEQ ID NO: 25), penetratin-RGD, or the like.

Targeting moieties useful for certain embodiments of the present invention are selected from, although they are not limited to, peptides, as for example, CendR peptides (e.g. Lyp1, cLyp1, tLyp1, iRGD, iNGR, TT1, linear TT1, RPARPAR, F3, etc.), RGD peptides, NGR peptides; proteins (e.g. transferrin, ankyrin repeat protein, insulin); small molecules (e.g. folic acid, triphenylphosphonium, ACUPA, PSMA); carbohydrate moieties (e.g. mannose, glucose, galactose and their derivatives); antibodies (including nanobodies, antibody fragments, monoclonal antibodies, affibodies or other antibodies) and aptamers.

In one embodiment the composition comprises a targeting moiety. In another embodiment the targeting moiety comprises a cell-penetrating peptide and/or a tumor/tissue-penetrating peptide. In a further embodiment, the targeting moiety is selected from the group consisting of Lyp-1, tLyp-1, and cLyp-1. In another embodiment, the polymer is PSA or HA and the targeting moiety is tLyp-1.

Bond between the polymer and the targeting moiety

In certain embodiments, some of the polymer (e.g., PSA, HA, PGA) is bonded to a targeting moiety, e.g., covalently. The polymer is bonded to a targeting moiety directly or indirectly e.g., via a linker, such as an aminoalkyl ($C_1$-$C_4$) succinimide linker (including $C_1$, $C_2$, $C_3$, and $C_4$), or aminoalkyl ($C_1$-$C_4$) amido-isopropyl linker (including $C_1$, $C_2$, $C_3$, and $C_4$). In some cases, other aminoalkylsuccinimide or aminoalkylamido-iso-propyl linkers are used. In some embodiments, the targeting moiety comprises a C terminus, e.g., for binding. In some cases, the aminoalkyl ($C_1$-$C_4$) succinimide linker is an aminoethylsuccinimide linker, an aminopropylsuccinimide, an aminobutylsuccinimide, or the like. The aminoalkyl ($C_1$-$C_4$) succinimide linker can be created, e.g., using an EDC/NHS (1-ethyl-3-(3-dimethylaminopropyl)carbodiimide hydrochloride/N-hydroxysuccinimide) coupling reaction to attach a maleimide moiety to a carboxylic acid moiety on a monomer unit (e.g., a sialic acid unit). In some cases, an N-aminoalkyl ($C_1$-$C_4$) maleimide moiety, such as an N-aminoethyl

maleimide moiety is reacted with a carboxylic acid moiety on a monomer unit to produce an amide bond, thereby joining the maleimide moiety to the polymer (e.g., PSA). The aminoalkyl ($C_1$-$C_4$) amido-iso-propyl linker can be created, e.g., using aminoethylmethacrylamide or N-(3-aminopropyl)methacrylamide in the presence of BOP/TBA (benzotriazol-1-yloxy-tris(dimethyl-amino) phosphonium hexafluorophosphate/tetra-n-butylammonium hydroxide).

The maleimide moiety or the methacryloyl moiety then can react, e.g., via Michael-type addition, with a cysteine, a thiol group or other sulfur-containing moiety within the peptide to bond the peptide to the polymer (e.g., PSA, HA, PGA) via an aminoalkyl ($C_1$-$C_4$) succinimide, such as aminoethylsuccinimide linker, or via an aminoalkyl ($C_1$-$C_4$) amido-iso-propyl linker.

In some embodiments, the polymer (e.g., PSA, HA, PGA) is bonded to a targeting moiety directly through an amide group. The amide group can be created, e.g., by reacting a carboxylic acid moiety on a monomer unit (e.g., a sialic acid unit) and a lysine, arginine or other primary amine-containing moiety within the peptide, in particular the primary amine group is in a lysine or arginine amino acid on the targeting moiety. In some embodiments an activator is present in the reaction to form an intermediate, as e.g., a carbodiimide, N-hydroxysuccinimide or DMTMM (4-(4,6-dimethoxy-1,3,5-triazin-2-yl)-4-methylmorpholinium chloride).

Thus, one set of embodiments is generally directed to a method of reacting a carboxylate moiety on a polymer (e.g., PSA, HA, PGA) with an aminoalkyl ($C_1$-$C_4$) maleimide and/or an aminoalkyl ($C_1$-$C_4$) methacrylamide, and reacting the resulting aminoalkyl ($C_1$-$C_4$) maleimide and/or the aminoalkyl ($C_1$-$C_4$) methacrylamide to a cysteine group on a peptide to produce polymer-aminoalkyl ($C_1$-$C_4$) succinimide-peptide and/or a polymer-aminoalkyl ($C_1$-$C_4$) amidoisopropyl-peptide composition.

Another set of embodiments is directed to a method of reacting a carboxylate moiety on a polymer (e.g., PSA, HA, PGA) with a N-hydroxysuccinimide or a carbodiimide, and reacting the intermediate formed with a lysine or arginine group on a peptide to produce a polymer-amide-peptide.

In one embodiment, the targeting moiety is bonded to the polymer electrostatically. In another embodiment, the targeting moiety is bonded to the polymer via a linker. In a particular embodiment, the polymer is PSA or HA and the targeting moiety is tLyp-1.

Polymer linked to hydrophobic moiety

The outer shell comprising e.g., PSA or HA, can be linked to a hydrophobic moiety covalently, electrostatically or by other means. The hydrophobic moiety may comprise an alkyl group, e.g. a straight-chain alkyl group. In some cases, the hydrophobic moiety comprises at least 2 carbon atoms. In other cases, the hydrophobic moiety comprises at least 3 carbon atoms. In some embodiments, the hydrophobic moiety comprises a $C_2$-$C_{24}$ straight-chain alkyl group (e.g., $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{16}$, $C_{16}$, $C_{17}$, $C_{18}$, $C_{19}$, $C_{20}$, $C_{21}$, $C_{22}$, $C_{23}$, and/or $C_{24}$). In a particular embodiment, the hydrophobic moiety comprises a straight-chain $C_{12}$ alkyl group. In some cases, the composition of the invention further comprises an aliphatic carbon chain covalently bonded to the polymer (e.g., PSA). In other cases, the aliphatic carbon chain comprises a $C_2$-$C_{24}$ aliphatic carbon chain (e.g., $C_2$, $C_3$, $C_4$, $C_5$, $C_6$, $C_7$, $C_8$, $C_9$, $C_{10}$, $C_{11}$, $C_{12}$, $C_{13}$, $C_{14}$, $C_{16}$, $C_{16}$, $C_{17}$, $C_{18}$, $C_{19}$, $C_{20}$, $C_{21}$, $C_{22}$, $C_{23}$, and/or $C_{24}$).

Other examples of hydrophobic moieties include $C_3$, $C_4$, $C_5$, $C_6$, $C_7$, $C_8$, $C_9$, $C_{10}$, $C_{11}$, $C_{12}$, $C_{13}$, $C_{14}$, $C_{15}$, $C_{16}$, $C_{17}$, $C_{18}$, $C_{19}$, $C_{20}$, $C_{21}$, $C_{22}$, $C_{23}$, $C_{24}$, or other alkyl group (e.g., a straight-chain or branched alkyl group, e.g., an isoalkyl group). In some cases, the hydrophobic moiety comprises at least 3, at least 4, at least 5, at least 6, at least 7, at least 8, at least 9, at least 10, at least 11, at least 12, at least 13, at least 14, at least 15, at least 16, at least 17, at least 18, at least 19, at least 20, at least 21, at least 22, at least 23, or at least 24 carbon atoms. The hydrophobic moieties are saturated or unsaturated, e.g., containing one or more carbon-carbon double or triple bonds. Some techniques for attaching hydrophobic moieties are described in WO2019086627A1. In some cases, hydrophobic moieties are attached using activation by a quaternary ammonium salt (e.g., tetrabutylammonium hydroxide) and a tetrafluoroborate (e.g., 2-bromo-1-ethyl pyridinium tetrafluoroborate), prior to reaction with a hydrophobic moiety (e.g., an alkyl amine, such as dodecylamine for $C_{12}$).

In one embodiment, at least some of the polymers are linked to a hydrophobic moiety. In another embodiment the hydrophobic moiety is selected from the group consisting of an alkyl group, cycloalkanes, bile salts and derivatives, terpenoids, terpenes, terpene-derived moieties and lipophilic vitamins. In a further embodiment, the hydrophobic moiety comprises a $C_2$-$C_{24}$ straight-chain alkyl group. In a still further embodiment, the hydrophobic moiety comprises a $C_{16}$ straight-chain alkyl group.

In a still further embodiment, the polymer is HA and is linked to a hydrophobic moiety. Particularly, the hydrophobic moiety comprises a $C_{16}$ straight-chain alkyl group. The nanoentities may comprise optionally a tLyp-1 targeting moiety.

Surfactants

The nanoentities may comprise one or more surfactants. Examples of surfactants include, but are not limited to,

the following: polyoxyethylene sorbitan monooleate (polysorbate 80; Tween 80®; HLB 15), polyoxyethylene sorbitan monostearate (Tween® 60, HLB 14.9 and Tween 61®; HLB 9.6), polyoxyethylene sorbitan monooleate (Tween 81®; HLB 10), polyoxyethylene sorbitan tristearate (Tween 65®; HLB 10.5), polyoxyethylene sorbitan trioleate (Tween 85®; HLB 11), polyoxyethylene sorbitan monolaurate (Tween® 20, HLB 16.7 and Tween 21®; HLB 13.3), polyoxyethylene sorbitan monopalmitate (Tween® 40, HLB 15.6); PEGylated fatty acid esters and mixtures with PEG, polyethylene glycol monostearate (HLB 11.6), polyethylene glycol stearate, polyethylene glycol stearate 40 (HLB 17), polyethylene glycol stearate 100 (HLB 18.8), polyethylene glycol dilaurate 400 (HLB 9.7), polyethylene glycol dilaurate 200 (HLB 5.9), polyethylene glycol monopalmitate (HLB 11.6), Macrogol 15 Hydroxystearate (Kolliphor HS15®, BASF), polyethylene glycol-15-hydroxystearate (HLB 14-16), D-alpha-tocopheryl polyethylene glycol succinate (TPGS; HLB 13.2), triethanolammonium oleate (HLB 12), sodium oleate (HLB 18), sodium cholate (HLB 18), sodium deoxycholate (HLB 16), sodium lauryl sulphate (HLB 40), sodium glycocholate (HLB 16-18), triethanolamine oleate (HLB 12), gum tragacanth (HLB 11 .9) and sodium dodecyl sulphate (HLB 40); Poloxamer 124 (HLB 16), Poloxamer 188 (HLB 29), Poloxamer 237 (HLB 29), Poloxamer 238 (HLB 28), Poloxamer 278 (HLB 28), Poloxamer 338 (HLB 27), and Poloxamer 407 (HLB 22), sorbitan monooleate (Span® 80, HLB 4.3), sorbitan monolaurate (Span® 20, HLB 8.6), sorbitan monostearate (Span® 60, HLB 4.7), sorbitan trioleate (Span® 85, HLB 1 .8), sorbitan sesquiolate (Span® 83, HLB 3.7), sorbitan monopalmitate (Span® 40, HLB 6.7), sorbitan isostearate (Span® 120, HLB 4.7), Lauroyl macrogolglycerides (e.g. Gelucire® 44/14, HLB 14 and Labrafil® M2130CS, HLB 4), Stearoyl macrogolglycerides (e.g. Gelucire® 50/13, HLB 13), Linoleoyl macrogolglycerides (e.g. Labrafil® M2125CS, HLB 4), Oleoyl macrogolglycerides (Labrafil® M1944CS, HLB 4), Caprylocaproyl macrogolglycerides (Labrasol®, HLB 14), lecithins (e.g. egg lecithin, soybean lecithin, non-GMO lecithin, rapeseed lecithin, sunflower lecithin, lysolecithin, etc), phospholipids (e.g. egg phospholipids, soybean phospholipids, synthetic phospholipids, hydrogenated phospholipids, PEGylated phospholipids, sphingolipids, phosphatidylcholine, lysophosphaditylcholine, phosphadidylethanolamine, phosphatidylserine, etc.), Phosal®, Phospholipon®, or any combination of any of these and/or other surfactants. In some cases, the surfactant is cationic, e.g., benzethonium choride, benzalkonium chloride, CTAB (hexadecyltrimethylammonium bromide), cetrimide, tetradecyltrimethylammonium bromide, dodecyltrimethylammonium bromide, or the like. In some cases, the cationic surfactant contains an ammonium salt, e.g., as a head group. For example, the head group comprises a primary, secondary, tertiary, or quaternary ammonium salt. In addition, it should be understood that such surfactants are not required in all embodiments.

In some embodiments, the surfactant is mainly located at the inter-phase of the inner core and the outer shell.

In a particular embodiment, the surfactant is polyoxyethylene sorbitan monooleate (e.g., Tween 80®). In a particular embodiment, the nanoentities contain polyoxyethylene sorbitan monooleate (e.g., Tween 80®) and macrogol 15 hydroxystearate (Kolliphor HS15®) as surfactants.

Methods for producing compositions of nanocapsules

Various aspects of the invention are also generally directed to systems and methods for producing compositions such as those described herein, e.g., nanocapsules, or other nanoentities.

Polymeric nanocapsules, such as e.g., PSA-based nanocapsules, HA-based nanocapsules, PGA-based nanocapsules, can be produced by a variety of techniques. One of them is a solvent displacement technique, involving the mixing of a polar solvent in a water phase. Another technique is a self-emulsification technique, which does not require the use of organic solvents. In some cases, this includes preparing an aqueous solution that comprises a polymer (e.g., PSA, HA, PGA, PGA-PEG, PASP, PASP-PEG, PLMA, PLA-PEG, etc) and optionally one or more water-soluble surfactants, preparing an oily solution (e.g., comprising an oil and one or more surfactants, and an organic solvent, etc.), and mixing the solutions together. It is believed that the nanocapsules are formed due to the interaction of polymer e.g., PSA, HA, PGA, PASP with a positively charged surfactant at the interphase of the oil-in-water emulsion. However, the presence of a cationic surfactant may not be necessary when using hydrophobically modified polymers, as the presence of alkyl chains/hydrophobic components promotes the polymer attachment by its insertion in the oily nanodroplet.

Pharmaceutical agents can be dissolved in the aqueous phase or in the oily phase before preparing the nanoentities or incubated with the pre-formed nanoentities. When the pharmaceutical agent is an antibody such as a monoclonal antibody, the antibody is particularly encapsulated by dissolving it in the aqueous phase or in the oily phase (if highly concentrated) before preparing the nanocapsules.

As an example, in one embodiment, this includes preparing an aqueous phase that comprises a polymer (e.g., PSA or HA); preparing a hydrophobic phase comprising a hydrophobic compound (e.g., comprising an oil and one or more surfactants, and optionally an organic solvent, etc.); adding an antibody or a fragment thereof which binds to an intracellular epitope of an activated mutated KRAS protein to the aqueous phase or, optionally to the hydrophobic phase if the antibody or the fragment is highly concentrated; and mixing the aqueous phase and the hydrophobic phase under stirring. This method can be called herein a "one-step method". In a particular embodiment the hydrophobic compound is an oil and the hydrophobic phase also comprises a surfactant. In another embodiment, the aqueous phase further comprises a surfactant. In another embodiment, the polymer is linked to a hydrophobic moiety.

In another case, the method includes preparing an oily solution (e.g., comprising an oil and one or more surfactants and optionally an organic solvent, etc.), and adding it to an aqueous phase (or adding the aqueous phase over the oily phase). The aqueous phase comprises a polymer (e.g., PSA or HA), and optionally contains one or more water-soluble surfactants. The solutions are mixed under stirring to form nanocapsules. Once the nanocapsules are formed, an additional aqueous phase that comprises an antibody or a fragment thereof is added under stirring to produce the antibody-loaded nanocapsules by incubation. This method can be called herein a "two-steps method".

If an organic solvent is added, in some cases it is completely or partially evaporated.

The polymer may also be functionalized with a targeting moiety such as a cell-penetrating peptide and/or a tumor/tissue-penetrating peptide or with a hydrophobic moiety such as a straight-chain alkyl group.

As an example, PSA can be linked to tLyp-1 by covalent binding between thiol groups of the peptide tLyp-1 and carboxylate groups of PSA. This synthetic approach used the heterobifunctional linker aminoethyl maleimide which allows, first, its incorporation through the amine group of the linker to carboxylate groups of PSA (using carbodiimide chemistry) and second, peptide binding through the addition of the thiol group of the peptide (cysteine residue) to the maleimide group of the linker (Michael type addition), following a 2-step process. This strategy allowed the preservation of the biologically active groups of tLyp-1 peptide. Furthermore, the substitution degree can be easily controlled.

Typically, purified polypeptides such as antibodies are only marginally stable in an aqueous state and undergo chemical and physical degradation resulting in a loss of biological activity during processing and storage. Additionally, peptide compositions in aqueous phase undergo hydrolysis, such as deamidation and peptide bond cleavage. These effects represent a serious problem for therapeutically active antibodies which are intended to be administered to humans within a defined dosage range based on biological activity. For that reason, it was tested whether the produced nanoentities could maintain functionality after lyophilization.

Thus, the present disclosure relates to a method for producing nanoentities, comprising an additional step of lyophilization, which may preserve them during storage. **In** some cases, it is not necessary to use cryoprotectants during lyophilization. In some cases, it is not necessary to dilute the colloidal system before lyophilization, since the nanoentities do not form aggregates during reconstitution of the lyophilizate. In some cases, it is possible to add one or more sugars, e.g., sugars that exert a cryoprotectant effect. Examples of cryoprotectants include, but are not limited to, the following: trehalose, glucose, sucrose, mannitol, maltose, polyvinyl pyrrolidone (PVP), glycerol, polyethylene glycol (PEG), propylene glycol, 2-methyl-2,4-pentanediol (MPD), raffinose, dextran, fructose, stachyose, or the like. In some cases, cryoprotectants or other additives have other effects, e.g., as buffers to control pH. In lyophilized form, the nanoentities are stored for long periods of time, and can be regenerated, e.g., by adding water.

Combination of antibody or fragment thereof with other pharmaceutical agents/drugs

In a particular embodiment, the antibody or the fragment thereof is in combination with other pharmaceutical agents/drugs. They may be located internally and/or on the surface of the nanoentities, depending on the embodiment.

The pharmaceutical agent may have pharmacological activity and/or enhance the effect of the antibody or the fragment thereof, and/or may have other direct effect in the diagnosis, cure, mitigation, treatment or prevention of disease.

Given the increased metabolic requirements of tumor cells, it is not surprising that certain tumor types have developed mechanisms to scavenge nutrients from both extracellular and intracellular sources. In particular, RAS-driven cancers have several different metabolic adaptations that allow them to recycle various metabolites. This serves two key purposes; 1) it provides metabolic flexibility and efficiency; and 2) it ensures adequate availability of biosynthetic precursors. Importantly, these scavenging pathways have become critical to the metabolism of these cancers and may provide therapeutic opportunities. Thus, in particular embodiments, the anti-KRAS antibodies are combined with, e.g., macroautophagy inhibitors such as chloroquines, glucose transporters (GLUTs) inhibitors, lactate dehydrogenase inhibitors, and glycolytic inhibitors.

KRAS is a central node in a complex network providing many opportunities for feedback loops that allow cancer to proliferate and survive. Therefore, in particular embodiments, the anti-KRAS antibodies are combined with other targeted therapies such as those towards IGFR, mTOR, RAF, MEK, PIK3, EFGR/ERBB2, SHP2 or immune checkpoint inhibitors to potentially exert a synergistic effect in terms of tumor regression, contribute to generate an anti-tumor immunity, or overcome resistance as compared to each drug alone.

In a particular embodiment, the further pharmaceutical agent is an anticancer drug, such as e.g. paclitaxel or docetaxel.

The pharmaceutical agents are present at up to approximately 50 wt% relative to the total dry weight of the components of the system. However, the appropriate proportion will depend on a variety of factors such as the pharmaceutical agents that is to be incorporated, the indication for which it is used, the efficiency of administration, etc. For example, in some cases, the pharmaceutical agents are present at up to such as approximately 10 wt%, or up to such as approximately 5 wt%, or up to such as approximately 2 wt%, or up to such as approximately 1 wt%, or up to such as approximately 0,5 wt%, or up to such as approximately 0,1 wt%. **In** certain embodiments, more than one pharmaceutical

agent is present, which can be dissolved in the same solution or separately, depending on the nature of the active pharmaceutical ingredient to be incorporated.

Pharmaceutical compositions and administration forms

It is also disclosed a method of administering a composition comprising the nanoentities and particularly the nanocapsules discussed herein to a living organism. When administered, the compositions of the invention are applied in a therapeutically effective amount as a pharmaceutically acceptable formulation. As used herein, the term "pharmaceutically acceptable" means that the formulation contains agents or excipients compatible with the form required for administration to a living organism, without causing deleterious effects. Any of the compositions of the present invention are administered to the living organism in a therapeutically effective dose. A "therapeutically effective" or an "effective" as used herein means that amount necessary to delay the onset of, inhibit the progression of, halt altogether the onset or progression of, diagnose a particular condition being treated, or otherwise achieve a medically desirable result. When administered to a living organism, effective amounts will depend on the particular condition being treated and the desired outcome. A therapeutically effective dose is determined by those of ordinary skill in the art. Some embodiments of the invention are generally directed to the use of a composition as disclosed herein for the preparation of a medicament.

Any medically acceptable method is used to administer the composition to the living organism. The administration is localized (i.e., to a particular region, physiological system, tissue, organ, or cell type) or systemic, depending on the condition to be treated. For example, the composition is administered orally, or through other techniques such as vaginally, rectally, buccally, pulmonary, topically, nasally, transdermally, intratumorally, intraperitoneally, through parenteral injection or implantation, via surgical administration, or any other method of administration where access to the target by the composition of the invention is achieved. Compositions suitable for oral administration are presented as discrete units such as hard or soft capsules, pills, sachets, tablets, troches, or lozenges, each containing a predetermined amount of the active compound. Other oral compositions suitable for use with the invention include solutions or suspensions in aqueous or non-aqueous liquids such as a syrup, an elixir, or an emulsion. In another set of embodiments, the composition is used to fortify a food or a beverage. Rectal administration can be used in some embodiments, e.g., in the form of an enema, suppository, or foam.

In one set of embodiments, the administration of the composition is parenteral, intratumoral, or oral. In some embodiments, the composition is administered by injection or infusion. In one embodiment, the injection is selected from intratumoral, intraperitoneal, subcutaneous, intramuscular, or intravenous injection. In another embodiment, the composition is administered through intrathecal injection or infusion.

In certain embodiments of the invention, the administration of a composition of the invention is designed so as to result in sequential exposures to a composition over a certain time period, e.g., hours, days, weeks, months, or years. This is accomplished, e.g., by repeated administrations of a composition of the invention by one of the methods described above. Administration of a composition can be alone, or in combination with other therapeutic agents and/or compositions.

In a particular embodiment, the administration of the composition is intravenously, intratumoral, intraperitoneal, subcutaneous or by inhalation. Particularly, the administration is intravenous.

In certain embodiments of the invention, a composition can be combined with a suitable pharmaceutically acceptable carrier, e.g., as incorporated into a polymer release system, or suspended in a liquid, e.g., in a dissolved form or a colloidal form. In general, pharmaceutically acceptable carriers suitable for use in the invention are well-known to those of ordinary skill in the art. As used herein, a "pharmaceutically acceptable carrier" refers to a non-toxic material that does not significantly interfere with the effectiveness of the biological activity of the active compound(s) to be administered, but is used as a formulation ingredient, e.g., to stabilize or protect the active compound(s) within the composition before use. The term "carrier" denotes an organic or inorganic ingredient, which is natural or synthetic, with which one or more active compounds of the invention are combined to facilitate the application of a composition as discussed herein. The carrier is co-mingled or otherwise mixed with one or more compositions of the present invention, and with each other, in a manner such that there is no interaction that would substantially impair the desired pharmaceutical efficacy. The carrier is either soluble or insoluble, depending on the application. Examples of well-known carriers include glass, polystyrene, polypropylene, polyethylene, dextran, nylon, amylase, natural and modified cellulose, polyacrylamide, agarose and magnetite. The nature of the carrier can be either soluble or insoluble. Those skilled in the art will know of other suitable carriers, or will be able to ascertain such, using only routine experimentation.

In some embodiments, a composition of the invention can include pharmaceutically acceptable carriers with formulation ingredients such as salts, carriers, buffering agents, emulsifiers, diluents, excipients, chelating agents, fillers, drying agents, antioxidants, antimicrobials, preservatives, binding agents, bulking agents, silicas, solubilizers, or stabilizers that are used with the active compound. For example, if the formulation is a liquid, the carrier may be a solvent, partial solvent, or non-solvent, and may be aqueous or organically based. Examples of suitable formulation ingredients include diluents such as calcium carbonate, sodium carbonate, lactose, kaolin, calcium phosphate, or sodium phosphate; granulating and disintegrating agents such as corn starch or alginic acid; binding agents such as starch, gelatin

or acacia; lubricating agents such as magnesium stearate, stearic acid, or talc; time-delay materials such as glycerol monostearate or glycerol distearate; suspending agents such as sodium carboxymethylcellulose, methylcellulose, hydroxypropylmethylcellulose, sodium alginate, polyvinylpyrrolidone; dispersing or wetting agents such as lecithin or other naturally-occurring phosphatides; thickening agents such as cetyl alcohol or beeswax; buffering agents such as acetic acid and salts thereof, citric acid and salts thereof, boric acid and salts thereof, or phosphoric acid and salts thereof; or preservatives such as benzalkonium chloride, chlorobutanol, parabens, or thimerosal. Suitable carrier concentrations can be determined by those of ordinary skill in the art, using no more than routine experimentation. A composition as discussed herein can be formulated into preparations in solid, semi-solid, liquid or gaseous forms such as tablets, capsules, elixirs, powders, granules, ointments, solutions, depositories, inhalants or injectables. Those of ordinary skill in the art will know of other suitable formulation ingredients, or will be able to ascertain such, using only routine experimentation.

Preparations include sterile aqueous or non-aqueous solutions, suspensions and emulsions, which can be isotonic with the blood of the living organism in certain embodiments. Examples of non-aqueous solvents are polypropylene glycol, polyethylene glycol, vegetable oil such as olive oil, sesame oil, coconut oil, peanut oil, mineral oil, injectable organic esters such as ethyl oleate, or fixed oils including synthetic mono or di-glycerides. Aqueous carriers include water, alcoholic/aqueous solutions, emulsions or suspensions, including saline and buffered media. Parenteral vehicles include sodium chloride solution, 1,3-butandiol, Ringer’s dextrose, dextrose and sodium chloride, lactated Ringer’s or fixed oils. Intravenous vehicles include fluid and nutrient replenishers, electrolyte replenishers (such as those based on Ringer’s dextrose), and the like. Preservatives and other additives are also present such as, e.g., antimicrobials, antioxidants, chelating agents and inert gases and the like. Those of skill in the art can readily determine the various parameters for preparing and formulating a composition as discussed herein without resort to undue experimentation.

The present disclosure also provides any of the above-mentioned compositions in kits, optionally including instructions for use of the composition for e.g. the treatment of cancer. Instructions also may be provided for administering a composition by any suitable technique as previously described, e.g., orally or intravenously. The kit typically defines a package including any one or a combination of compositions of the invention and other ingredients as previously described. The kits also can include other containers with one or more solvents, surfactants, preservative and/or diluents (e.g., normal saline (0.9% NaCl), or 5% dextrose) as well as containers for mixing, diluting or administering the composition to a living organism. The compositions of the kit may be provided as liquid solutions or as dried powders. When a composition provided is a dry powder, the composition may be reconstituted by the addition of a suitable solvent. In embodiments where liquid forms of a composition are used, the liquid form may be concentrated or ready to use. The solvent will depend on a composition and the mode of use or administration.

Medical applications

An aspect of the invention relates to the composition of nanoentities for use in medicine (i.e. as a medicament). One aspect of the invention relates to the composition comprising the nanoentities for use in the prevention and/or treatment of a disease associated with a mutation in a KRAS gene. Alternatively, the disclosure relates to the use a composition of the invention for the preparation of a medicament for the prevention and/or treatment of a disease associated with a mutation in a KRAS gene. Alternatively, the disclosure relates to a method for preventing and/or treating a disease associated with a mutation in a KRAS gene comprising administering a composition of the invention to a subject in need thereof.

As used herein the terms "treat, " "treatment, " or "treatment of" refers to reducing the potential for a certain disease or disorder, reducing the occurrence of a certain disease or disorder, and/or a reduction in the severity of a certain disease or disorder, particularly, to an extent that the subject no longer suffers discomfort and/or altered function due to it. For example, "treating" can refer to the ability of a therapy when administered to a subject, to prevent a certain disease or disorder from occurring and/or to cure or to alleviate certain disease symptoms, signs, or causes. "Treating" also refers to mitigating or decreasing at least one clinical symptom and/or inhibition or delay in the progression of the condition and/or prevention or delay of the onset of a disease or illness. Thus, the terms "treat," "treating" or "treatment of" (or grammatically equivalent terms) refer to both prophylactic and therapeutic treatment regimes. Particularly, "treatment", as used herein, relates to the administration of a composition according to the invention to a subject suffering from a disease associated to a mutation in a KRAS gene, including the administration in an initial or early stage of a disease, wherein the object is to prevent or slow down (lessen) an undesired physiological change or disorder. The present disclosure provides uses and compositions generally providing a therapeutic benefit or desired clinical results. A therapeutic benefit is not necessarily a cure for a particular disease or disorder, but rather encompasses a result which most typically includes alleviation of the disease or disorder or increased survival, elimination of the disease or disorder, reduction or alleviation of a symptom associated with the disease or disorder, prevention or alleviation of a secondary disease, disorder or condition resulting from the occurrence of a primary disease or disorder, diminishment of extent of disease, stabilized (i.e., not worsening) state of disease, delay or slowing of disease progression, amelioration or palliation of the disease state, and remission

(whether partial or total), whether detectable or undetectable and/or prevention of the disease or disorder. Treatment also means prolonging survival as compared to expected survival if not receiving the treatment.

The term "prevention", "preventing" or "prevent", as used herein, relates to the administration of a combination according to the invention or of a pharmaceutical composition according to the invention to a subject who has not been diagnosed as possibly having a disease associated to a mutation in a KRAS gene at the time of administration, but who would normally be expected to develop said disease or be at increased risk for said disease. The prevention intends to avoid the appearance of said disease. The prevention can be complete (e.g. the total absence of a disease). The prevention can also be partial, such that e.g. the occurrence of a disease in a subject is less than that which would have occurred without the administration of the composition of the present invention. Prevention also refers to reduced susceptibility to a clinical condition.

The term "subject" or "individual" or "animal" or "patient" includes any subject, particularly a mammalian subject, for whom diagnosis, prognosis, or therapy is desired. Mammalian subjects include humans, domestic animals, farm animals, and zoo or pet animals such as dogs, cats, guinea pigs, rabbits, rats, mice, horses, cattle, cows, and so on. In a particular embodiment of the invention, the subject is a mammal. In a more particular embodiment of the invention, the subject is a human, particularly a human of any race and sex. In some embodiments, a subject is a naive subject. A naive subject is a subject that has not been administered a therapy. In another embodiment, a subject has received therapy and/or one or more doses of a therapeutic agent to treat the disease associated to a mutation in a KRAS gene.

Oncogenic RAS mutations contribute to the induction of various cancers including pancreatic ductal adenocarcinomas (PDACs), colorectal adenocarcinomas (CRCs), lung adenocarcinomas, and gastric adenocarcinomas. Mutated RAS genes, representing the most frequently mutated oncogene family, are present in approximately 25% of human tumors. Mutations within KRAS account for 85% of all RAS family oncogenic mutations.

In a particular embodiment, the disease associated to a mutation in KRAS gene is cancer. In more particular embodiments, cancer is a hematological malignancy such as leukemia. The term "hematological malignancy" refers to a type of cancer that affects blood, bone marrow, and lymph nodes, and includes lymphomas, myelomas and leukemias. Examples of hematological malignancies wherein KRAS gene is mutated are acute myelogenous leukemia (AML), core binding factor acute myeloid leukemia, juvenile myelomonocytic leukemia (JMML), multiple myeloma, myelodysplastic syndromes and autoimmune lymphoproliferative syndrome.

In other particular embodiments, cancer is a solid tumor. "Solid tumor" or solid cancers are neoplasms (new growth of cells) or lesions (damage of anatomic structures or disturbance of physiological functions) formed by an abnormal growth of body tissue cells other than blood, bone marrow or lymphatic cells. A solid tumor consists of an abnormal mass of cells which may stem from different tissue types such as liver, colon, breast, or lung, and which initially grows in the organ of its cellular origin. However, such cancers may spread to other organs through metastatic tumor growth in advanced stages of the disease. Examples of solid tumors are carcinomas, sarcomas, germinomas and blastomas.

In particular embodiments, cancer is an adenocarcinoma. In some embodiments, cancer is gastric cancer (particularly gastric adenocarcinoma), cholangiocarcinoma, lung cancer (particularly lung adenocarcinomas), colorectal cancer (particularly colorectal adenocarcinoma (CRCs)), pancreatic cancer (particularly ductal adenocarcinoma (PDACs)), skin cutaneous melanoma, uterine corpus endomietriod carcinoma, uterine carcinosarcoma, thyroid carcinoma, stomach adenocarcinoma, bladder urothelial carcinoma, cercical adenocarcinoma, head and neck squamous cell carcinoma, esophageal adenocarcinoma.

G12V and G12D KRAS substitutions are among the most commonly observed mutations in pancreatic adenocarcinoma (30% and 51%, respectively) and colorectal adenocarcinomas (27% and 45%, respectively) and have been associated with poor prognosis. Thus, in particular embodiments, cancer is pancreatic adenocarcinoma and colorectal adenocarcinoma.

In a particular embodiment, the disease is associated with a substitution in G12 of a KRAS gene. More particularly, the disease is non-small cell lung carcinoma, colorectal carcinoma, malignant solid tumor, acute myeloid leukemia, squamous cell lung carcinoma, colorectal adenocarcinoma, pancreatic ductal adenocarcinoma, rectal adenocarcinoma, small cell lung carcinoma, glioma, thyroid gland adenocarcinoma, multiple myeloma and myelodysplastic syndromes.

In a particular embodiment, the substitution is G12V and the disease is colorectal adenocarcinoma, non-small cell lung carcinoma, pancreatic ductal adenocarcinoma, uterine corpus neoplasm, and ovarian neoplasm, which have the greatest prevalence.

In a particular embodiment, the substitution is G12C and the disease is non-small cell lung carcinoma, colorectal adenocarcinoma, adenocarcinoma of unknown primary, uterine corpus neoplasm, and cancer of unknown primary, the above-mentioned having the greatest prevalence.

In a particular embodiment, the substitution is G12D and the disease is colorectal adenocarcinoma, pancreatic ductal adenocarcinoma, non-small cell lung carcinoma, uterine corpus neoplasm, and ovarian neoplasm having the greatest prevalence.

In other embodiments, the disease associated to a KRAS mutation is Noonan syndrome (NS), Cardiofaciocutaneous syndrome (CFC), and epidermal nevus. Germline mutations in the KRAS gene also cause a disorder whose major features overlap with those of cardiofaciocutaneous syndrome and two related disorders called Noonan syndrome and Costello syndrome. This condition has been described as the KRAS mutation-associated phenotype.

The present disclosure is directed to nanoentities for drug delivery applications. For example, such nanoentities are delivered into a subject such that they reach a tumor that the subject is suffering from. The nanoentities are delivered into the tumor cells, optionally facilitated by a targeting moiety which also have capacity as cell- or tumor/tissue-penetrating peptides such as Lyp-1 or tLyp-1, or other peptides discussed herein. Once delivered, the nanoentities can access the target cells, e.g. cancer cells and metastatic cancer cells, and release the drug contained therein (i.e. the anti-KRAS antibody). Thus, the present disclosure relates to a method of delivering an anti-KRAS antibody or a fragment thereof to target cells comprising administering to a subject a composition comprising the nanoentities according to the invention, wherein the nanoentities cross the cell membranes of the target cells. In particular, the target cells are cancer cells. According to the examples provided herein, the nanoentities are able to penetrate the cells causing the intracellular delivery of the anti-KRAS antibody or fragment thereof. Thus, the present disclosure relates to the composition according to the invention for intracellular delivery, in particular, for *in vivo* intracellular delivery. Further, the nanoentities with the antibody or the fragment thereof are able to perform their biological function inside the cell, e.g. the reduction of cell proliferation. Thus, the present disclosure also provides a method for reducing cell proliferation in a tumor cell, comprising administering to a subject a composition comprising the nanoentities according to the invention.

Unless otherwise defined, all technical and scientific terms used herein have the same meaning as commonly understood by a person skilled in the art. Methods and materials similar or equivalent to those described herein can be used in the practice of the present invention. Throughout the description and claims the word "comprise" and its variations such as "comprising" are not intended to exclude other technical features, additives, components, or steps. Additional objects, advantages and features of the invention will become apparent to those skilled in the art upon examination of the description or may be learned by practice of the invention. Furthermore, the present invention covers all possible combinations of particular and preferred embodiments described herein. The following examples and drawings are provided herein for illustrative purposes, and without intending to be limiting to the present invention.

## EXAMPLES

### EXAMPLE 1: Ability of the anti-KRAS mAbs to specifically interact with their target proteins

This example illustrates the ability of the anti-KRAS monoclonal antibodies to specifically interact with their target proteins. Two different studies were performed to investigate this ability:

(i) Interaction of anti-KRAS monoclonal antibodies with the synthetic antigenic peptides used for their former generation by hybridoma technology. These peptides correspond to the amino acid sequence (positions 5-16) of the KRAS protein. The peptides were synthesized according to the sequences depicted in Table 2 (Karebay Biochem, USA; >95% purity according to CoA) and further conjugated to a carrier protein, bovin serum albumin (BSA), for practical reasons (Biogenes, Germany). The internal codes assigned to the peptides once conjugated to BSA are also shown in Table 2 below.

(ii) Interaction of anti-KRAS monoclonal antibodies with different recombinant human mutated KRAS proteins.

(i) Interaction of anti-KRAS monoclonal antibodies with the synthetic antigenic peptides

Preparation of the peptide-BSA conjugate for ELISA testing: A terminal cysteine residue was added to the peptide during its synthesis and the sulfhydryl group was used to conjugate the peptide to BSA by the following two-step method:

1. Maleylation of BSA: 1 mg of the crosslinker SMCC (50 mg/ml, in N-methyl-2-pyrrolidone, NMP) was added to 1 ml of BSA solution (10 mg/ml, in 0.1 mM $NaHCO_3$, pH 8.3). After incubation for 1 h at room temperature (RT), the solution was desalted using a Sephadex G-50 - column (1.5 x 14 cm) previously equilibrated with PBS.

2. Conjugation of the maleylated BSA: 50 μl of the peptide solution (10 mg/ml, in doubly distilled water) was added to 1 ml of maleylated BSA (1.0 mg/ml, in PBS) and incubated at RT for 2 h at 4 °C followed by another 4 h at RT. Unreacted maleimide groups were blocked by addition of 2-mercaptoethanol to a final concentration of 10 mM and overnight incubation at 4 °C. Finally, the conjugate was dialyzed against 3 x 500 volumes of PBS at 4 °C (MW-cut-off 10,000).

Table 2

| KRAS mutation | Peptide sequence | Code |
|---|---|---|
| G12V | KLVVVGA**V**GVGK (SEQ ID NO: 40) | 820940-BSA |
| G12D | KLVWGA**D**GVGK (SEQ ID NO: 41) | 820941-BSA |
| G12C | KLVWGA**C**GVGK (SEQ ID NO: 42) | 820942-BSA |

ELISA plates were coated with antigenic peptide 820940-BSA (G12V), antigenic peptide 820941-BSA (G12D) and antigenic peptide 820942-BSA (G12C) at 4 μg/ml (50 μl/well) and processed employing an alkaline phosphatase-mediated indirect ELISA using a goat anti-mouse IgG Fc-specific as a secondary antibody. The results represent the $OD_{405nm}$ after 15 min of substrate incubation.

All anti-KRAS antibodies (clone DWP, clone D113 and clone D210) interacted with their corresponding antigenic peptides. Results obtained, for clones D113 and D210 titrated with their corresponding antigenic peptide 820941-BSA (G12D) are represented in Table 3 below.

Table 3:

| Antibody concentration (ng/mL) | D113 clone | D210 clone |
|---|---|---|
| 625 | 2.265 | 1.960 |
| 156 | 2.239 | 1.916 |
| 39 | 2.228 | 1.760 |
| 9.8 | 1.997 | 1.648 |
| 2.4 | 1.762 | 1.453 |
| 0.6 | 1.394 | 1.140 |
| Blank* | 0.009 | |
| *Blocking buffer instead of antibody for sample preparation | | |

(ii) Interaction of anti-KRAS monoclonal antibodies with different recombinant human mutated KRAS proteins.

Next an ELISA plate was coated with a recombinant KRAS (G12V) mutated protein (SignalChem, Canada, catalog no. R06-32CH) at 2 μg/ml (50 μl/well) and processed employing an alkaline phosphatase-mediated indirect ELISA using a goat anti-mouse IgG Fc-specific as a secondary antibody. The results represent the $OD_{405nm}$ after 15 min of substrate incubation.

An ELISA plate was also coated with a recombinant KRAS (G12D) mutated protein (Sino Biological, catalog no. 12259-H07E1) at 5 μg/mL (50 μl/well) and processed employing a horseradish peroxidase-mediated indirect ELISA using a goat anti-mouse IgG (H+L), HRP conjugate as a secondary antibody. The results represent the $OD_{405\ nm}$ after 25 min of substrate incubation.

All anti-KRAS antibodies (clone DWP, clone D113 and clone D210) interacted with their corresponding mutated target proteins.

Additionally, the specificity of the anti-KRAS antibodies (subclones DWP, D113 and D210) was tested using different antigenic peptides corresponding to G12V, G12D, and G12C KRAS mutations (4 ug/mL) and also with the recombinant G12V KRAS protein (5 μg/ml).

As it is shown in Table 4 below, each clone was only able to interact with its corresponding target peptide and/or protein.

Table 4:

| Clone | Peptide 820940-BSA (G12V) | Peptide 820941-BSA (G12D) | Peptide 820942-BSA (G12C) | Recombinant KRAS G12V protein |
|---|---|---|---|---|
| DWP (G12V) | 0.841 | 0.046 | 0.054 | 2.769 |
| D113 (G12D) | 0.006 | 2.391 | 0.005 | 0.053 |
| D210 (G12D) | 0.007 | 2.099 | 0.007 | 0.020 |

Conclusions:

The anti-KRAS antibodies (clone DWP, D113 and D210) were able to selectively interact with the corresponding antigenic peptides (used for their generation by hybridoma technology) and also with the corresponding recombinant human KRAS target proteins. Therefore, DWP only interacted with 820940-BSA peptide (G12V) and with the recombinant KRAS G12V protein; whereas D113 and D210 only interacted with 820941-BSA peptide (G12D) and with the recombinant KRAS G12D protein.

**EXAMPLE 2: Determination of the KD value of two anti-KRAS mabs (D210 and D113) for their soluble antigen (KRAS G12D protein)**

This example illustrates the determination of the KD value of two anti-KRAS monoclonal antibodies (D210 and D113) for their soluble antigen (KRAS G12D protein) via Surface Plasmon Resonance (SPR) technology (GE Biacore 8K).

KD is the equilibrium dissociation constant, which is inversely related to affinity: the lower the KD value the higher is the affinity of the antibody. Antibody affinity refers to the strength with which the epitope binds to an individual paratope (antigen-binding site) on the antibody. High affinity antibodies bind quickly to the antigen and maintain this bond more readily under different conditions. Most antibodies have $K_D$ values in the low micromolar ($10^{-6}$) to nanomolar ($10^{-7}$ to $10^{-9}$) range. High affinity antibodies are generally considered to be in the low nanomolar range ($10^{-9}$).

Briefly, the KRAS G12D protein at a defined concentration was flown over the anti-KRAS antibody-coated sensor chip (protein A sensor chip) and response was captured over time, showing the progress of the interaction and association/dissociation cycle. The response measures changes in refractive index and is related to variations in mass close to the sensor surface. Therefore, the response is proportional to the number of antigen molecules interacting with the antibodies. After different concentrations of KRAS G12D protein were successively tested (regeneration is performed to remove all remaining bound protein from the chip from one concentration to another), the kinetics parameters and affinity were calculated using BIA-evaluation software. KD (M) of anti-KRAS antibodies D113 and D210 were $4.98 \times 10^{-9}$ and $3.75 \times 10^{-9}$, respectively.

Conclusion:

Both anti-KRAS G12D antibodies, D210 and D113, showed similar KD values in the low nanomolar range, which is indicative of a high affinity for the target (KRAS G12D protein).

**EXAMPLE 3: Formulation of different polymeric nanocapsules for the efficient association and delivery of anti-KRAS mabs**

This example illustrates the formulation of different polymeric nanocapsules for the efficient association and delivery of anti-KRAS monoclonal antibodies (mAbs). The polymer-forming shells can be prepared from biodegradable polyacids, which can be further functionalized, covalently or electrostatically, with targeting and/or tumor/tissue-penetrating ligands as, e.g., tLyp-1. Below, the preparation of functionalized polymers either by covalent attachment or simple ionic interaction of the targeting ligand tLyp-1 is described. Secondly, the preparation and characterization of nanocapsules loaded with anti-KRAS mAb and made of different polymers is detailed.

Preparation of covalently functionalized PSA polymer (PSA-tLyp-1)

Polysialic acid (PSA, 28 kDa, Serum Institute of India) was modified with N-(2-aminoethyl) maleimide trifluoroacetate salt using the following molar ratio N-(3-dimethylaminopropyl)-N'-ethylcarbodiimide hydrochloride (EDC)/ N-hydroxysuccinimide (NHS)/ N-(2-aminoethyl) maleimide trifluoroacetate salt (AEM)/tLyp-1: 11.6/2/0.40/0.0283. For this purpose, PSA was dissolved in 0.1 M MES buffer at pH 6 at a final concentration of 2 mg/mL, and the corresponding amount of EDC, NHS, and AEM were also dissolved in 0.1 M MES buffer, added to the PSA solution, and maintained under magnetic stirring for 4 hours at room temperature. The maleimide functionalized PSA (PSA-Mal) was purified by dialysis (regenerated cellulose, SnakeSkin 7 KDa MWCO, Thermo Scientific), first against NaCl 50 mM, and then against water. For the second reaction, PSA-Mal was dissolved in a solution of 0.1 M MES buffer and NaCl 50 mM at a final PSA concentration of 1 mg/mL. The peptide was added to this solution and the reaction mixture was maintained for 4 h under magnetic stirring at room temperature, and the final PSA-tLyp-1 product was purified by dialysis as described previously, freeze-dried, and stored at 4°C.

Preparation of electrostatically functionalized PSA and C16-HA polymers (PSA+tLyp-1; C16-HA+tLyp-1)

Polysialic acid (PSA, 28 kDa, Serum Institute of India) and C16-HA (Mw of 216 kDa and alkyl substitution degree of 5%, Contipro) were electrostatically modified with the targeting ligand tLyp-1. First, PSA or C16-HA were dissolved in 0.1 M MES buffer at pH 6 at a final concentration of 1 mg/mL and the corresponding amount of peptide tLyp-1 was added to this solution. The reaction mixture was maintained for 4 hours under magnetic stirring at room temperature, and the final products were purified by dialysis as described previously, freeze-dried, and stored at 4°C.

Preparation of polymeric nanocapsules

Nanocapsules with a polymer coating of PSA (28 kDa, Serum Institute of India), or PSA-tLyp-1, or HA (290 kDa, Lehvoss Iberica), or C16-HA (Mw of 216 kDa and alkyl substitution degree of 5%, Contipro), or C16-HA+tLyp-1, were prepared by a self-emulsifying technique. First, 59 mg Polysorbate 80 (Tween 80®, Merck) and 58 mg caprylic/capric triglycerides (Mygliol ® 812N, IOI Oleochemical GmbH) were weighted in a glass vial of 2 mL capacity (oily phase). Then, for those formulations containing non-hydrophobically modified or non-amphiphilic polymers as shells, a cationic surfactant was added to the oily phase (4 microliters of benzethonium chloride previously solubilized in ethanol, 50 mg/mL), otherwise it is not essential as the presence of alkyl chains/hydrophobic components promotes the polymer attachment by its insertion in the oily nanodroplet. All components of the oily phase were kept under magnetic stirring (500 rpm). In parallel, the aqueous phase was prepared by solubilizing, separately, each polymer at variable concentrations (e.g., for PSA-based formulations at 3 mg/mL, for HA-based formulations at 0.25-0.5 mg/mL), and Macrogol 15 Hydroxystearate (Kolliphor HS15®, BASF) in PBS pH 7.3 25 mM at a concentration of 20 mg/mL. After that, 0.75 mL of the polymer solution were conveniently mixed with 125 microliters of the Kolliphor solution and this aqueous phase was added over the oily phase under magnetic stirring (1100 rpm).

mAb association process used to associate the anti-KRAS mAbs anti-G12V (DWP) and anti-G12D (D113 and D210)

The required volume of anti-KRAS mAb to get the desired final mAb concentration (for instance 1, 2 or 3 mg/mL), was added to the aqueous phase before being mixed with the oily phase.

Physico-chemical characterization

The nanocapsules were characterized in terms of mean particle size and polydispersity index (PI) by photon correlation spectroscopy (PCS). Samples were diluted in MilliQ Water and the analysis was carried out at 25°C with an angle detection of 173°C. Zeta potential measurements were performed by laser Doppler anemometry (LDA) and the samples were diluted in ultrapure water. PCS and LDA analysis were performed in triplicate using a NanoZS® (Malvern Instruments, Malvern, UK). As otherwise stated, results corresponding to 3 replicates are shown in Table 5 and 6.

Association efficiency. To determine the association of the anti-KRAS mAbs to the nanocapsules, an aliquot of each different formulation was first diluted in PBS and then filtered (1 mL) in Amicon Stirred Cells® (polyethersulfone Biomax® 500 KDa Ultrafiltration Discs, Merck) at 4°C under 1 bar nitrogen pressure. The association efficiency was indirectly calculated as: [(total mAb - free mAb)/Total mAb]*100. The results obtained for anti-KRAS G12V and anti-KRAS G12D are shown in table 5 and 6, respectively, below.

Table 5:

| Formulation | mAb final concentration (mg/mL) | Size (nm) | PI | Z Potential (mV) | % mAb associated |
|---|---|---|---|---|---|
| C16-HA 216 KDa SD5% | 1 | 131±10 | 0.28 | -13±2 | 67±3 |
| C16-HA 216 KDa SD5% | 1.66 | 147±12 | 0.20 | -7±2 | 82±2 |
| C16-HA+tLyp-1 216 KDa SD5%* | 1.66 | 179±3 | 0.31 | -5±3 | n.d |
| HA 290 KDa | 2 | 139±6 | 0.29 | -6±3 | n.d |
| HA 290 KDa | 3 | 151±7 | 0.29 | -3±3 | n.d |
| PSA 28 KDa | 1 | 165±18 | 0.27 | -7±1 | n.d |
| tLyp-PSA 28 KDa * | 1 | 160±4 | 0.25 | -6±2 | n.d |
| tLyp-PSA 28 KDa | 2 | 136±15 | 0.28 | -6±2 | n.d |

(continued)

| Formulation | mAb final concentration (mg/mL) | Size (nm) | PI | Z Potential (mV) | % mAb associated |
|---|---|---|---|---|---|
| PSA+tLyp-1 28 KDa * | 1 .66 | 177±6 | 0.36 | -2±2 | n.d |
| n.d: not determined; PI: polydispersity index; *n=2 | | | | | |

Table 6:

| Formulation | Anti-KRAS G12D mAb | mAb final concentration (mg/mL) | Size (nm) | PI | Z Potential (mV) | % mAb associated |
|---|---|---|---|---|---|---|
| HA 290 KDa | D210 | 1 | 145±5 | 0.24 | -1±1 | 60±17 |
| HA 290 KDa | D113 | 1 | 131±5 | 0.28 | -8±1 | 59±13 |
| C16-HA KDa SD5% | | 1.5 | 139±4 | 0.27 | -8±0 | n.d |
| PSA 28 KDa | | 1.5 | 175±2 | 0.29 | -5±0 | 54±5 |
| n.d: not determined; PI: polydispersity index | | | | | | |

Freeze-drying studies

Preliminary freeze-drying studies were performed to assess the possibility to process anti-KRAS mAb-containing nanocapsules suspensions as powders for long-term storage. PSA anti-KRAS mAb-loaded nanocapsules were prepared by the method explained above (10 mL-batch), and a concentrated solution of trehalose was added to the nanocapsules suspension (final concentration of trehalose 10% w/v) prior to freeze-drying. The characteristics of the freeze-dried nanocapsules were analyzed, before and after freeze-drying (FD) at different reconstitution time points during storage at 4°C, by measuring particle size, PI, pH, Zeta potential, association efficiency and total mAb content (by ELISA). The measurements were done in the same way as described above. Results corresponding to 3 replicates are depicted in Table 7 below, where it is shown that no significant changes were produced in terms of physico-chemical properties and % mAb, either associated to the nanocapsules or just present in the formulation.

Table 7:

| Formulation | | Size (nm) | pH | PI | Z Potential (mV) | % mAb associated | % total mAb |
|---|---|---|---|---|---|---|---|
| PSA 28 kDa | Before FD | 100±1 | 7.30 | 0.22 | -7±1 | 63±5 | 101±8 |
| | After FD | 117±5 | 7.29 | 0.22 | -5±1 | 53±10 | 105±1 |
| | 1 month | 114±2 | 7.29 | 0.18 | -7±1 | 47±8 | 95±12 |
| | 2 months | 114±4 | 7.21 | 0.21 | -4±4 | 47±9 | n.d |
| FD: freeze-drying; n.d: not determined; PI: polydispersity index | | | | | | | |

Conclusions: Anti-KRAS monoclonal antibodies were efficiently associated (55-80%) to different polymeric nanocapsule compositions. The nanocapsules presented adequate physicochemical characteristics. The possibility of being processed as powders for long-term storage by freeze-drying was also demonstrated.

**EXAMPLE 4: Stability in plasma of different anti-KRAS mAb-loaded polymeric nanocapsules**

Often, the lack of efficacy of nanocarriers is a result of their aggregation in complex media. This may result from the high ionic strength and/or the presence of proteins in biological media. Thus, the stability in plasma of different anti-KRAS mAb-loaded polymeric nanocapsules was studied. Their stability was taken as an indicator of their potential for parenteral administration of the mAbs.

Stability in plasma. Anti-KRAS G12V loaded nanocapsules prepared as described in Example 3 were incubated in human plasma (dilution 1:10, at 37°C) under horizontal shaking (300 rpm, Heidolph Instruments GmbH & Co.). At predetermined times, samples of the incubation milieu were withdrawn for the analysis of particle size by a Malvern Zeta-Sizer and for the analysis of size and size distribution by Nanoparticle Tracking Analysis (NTA). Samples were analyzed after an appropriate further dilution (1:10.000 in PBS pH 7.4 10 mM for NTA; 1:1000 in water for dynamic light scattering (DLS)).

The stability of one representative formulation (anti-KRAS mAb-loaded HA nanocapsules, 0.5 mg/mL mAb concentration) measured by DLS and NTA is shown in FIGs. 1 and 2, respectively.

Conclusion: mAb-loaded nanocapsules showed an adequate stability in human plasma during at least 24 h, which represents an important benefit to be parenterally administered to a subject.

**EXAMPLE 5: *In vitro* cell internalization of anti-KRAS G12V mAb-loaded nanocapsules in colon adenocarcinoma cells expressing KRAS G12V mutation**

This example illustrates the cell internalization of the anti-KRAS G12V mAb in a SW480 colon adenocarcinoma cell line expressing the KRAS G12V mutation. The antibody was first labeled with a fluorescent marker (Alexa Fluor® 488) and, then, associated to the nanocapsules. The maintenance of the nanocapsules properties after loading of the fluorescent antibody was assessed.

Anti-KRAS mAb Labeling with Alexa Fluor® 488

Briefly, the mAb was diluted to a concentration of 2 mg/mL in 1x PBS (pH 7.2-8.0). Then, 50 μL of 1M sodium bicarbonate buffer were added to 0.5 mL of mAb solution (2 mg/mL) (final pH must be between 7.5 and 8.0). The solution was then added to a vial of the specific reactive dye supplied with the Alexa Fluor® 488 Protein Labeling Kit (ThermoFisher) and stirred for 1 hour at room temperature. After that the solution was kept overnight under 2-8°C. The purification column/resin was prepared by adding the resin inside a plastic column, supplied with the kit, up to around 3 cm from the top, and the excess of buffer was drained before loading the labeled protein (once at room temperature). When all the solution penetrated the resin, 1x PBS was added to elute the protein. The fraction containing the labeled protein (placed at the bottom) was then collected and protein concentration (M) and the Degree of labeling were calculated according to Equation 1 and 2, respectively:

$$\text{Protein concentration(M)} = \frac{\left[[\text{A280} - (\text{A494x0.11})]\text{x dilution factor}\right]}{203{,}000}$$

Where 203,000 $cm^{-1}M^{-1}$ is the molar extinction coefficient of a typical IgG and 0.11 is the correction factor to account for absorption of the dye at 280 nm.

$$\text{Moles dye per mole protein} = \frac{\left[[\text{A494}]\text{x dilution factor}\right]}{71{,}000\text{xprotein concentration (M)}}$$

Where 71,000 $cm^{-1}M^{-1}$ is the approximate molar extinction coefficient of the Alexa Fluor®488 dye at 494 nm.

Labeling degree resulted to be 5 moles of Alexa Fluor® 488 dye per mole of mAb, which is within the optimal range according to the kit specifications (4-9 moles of Alexa Fluor®488 dye per mole of mAb).

Preparation of nanocapsules containing a fluorescence-labeled anti-KRAS G12V mAb

For these studies, polymeric nanocapsule formulations were prepared as described above (Example 3) using the anti-KRAS G12V mAb previously labeled with Alexa Fluor® 488 (final mAb concentration: 1 mg/mL). Characterization results in terms of size, polydispersity and zeta potential are depicted in Table 8.

Table 8:

| Formulation | Size (nm) | PI | Zeta pot. (mV) |
|---|---|---|---|
| HA 290 KDa* | 143 ± 1 | 0.25 | -1 ± 1 |
| PSA 28 KDa* | 122 ± 1 | 0.24 | -3 ± 1 |
| *n=2 | | | |

*In vitro* cell internalization study

An Imaging Flow Cytometer (ImageStream®) was used to investigate the ability of the nanocapsules to elicit an effective internalization of the associated anti-KRAS antibody into the cells. Briefly, Alexa Fluor® 488-loaded nanocapsules were incubated in 24-well plates with SW480 cells, using separate wells per each time point to be studied (e.g. 0, 4 h, and 8 h). At each predetermined time point the cells were trypsinized and the images were acquired in the ImageStream® device. The effective internalization was determined by labelling cytoplasm acidic organelles with Lysotracker® fluorescent marker for live cells, and further confirmed by confocal microscopy (data not shown). Representative images are shown in FIG. 3.

Conclusion: Polymer (HA) NCs enabled the efficient internalization of a fluorescence labeled anti-KRAS G12V mAb in SW480 cells expressing KRAS G12V mutation. The internalized antibody showed a preferential localization at the target site, i.e., at the inner side of the plasma membrane, where oncogenic KRAS is localized.

**EXAMPLE 6. Effects of *in vitro* cell internalization of anti-KRAS G12V mAb-loaded nanocapsules in lung adenocarcinoma cells expressing KRAS G12V mutation**

This example illustrates the effects of anti-KRAS G12V mAb-loaded polymeric nanocapsules on the inhibition of cell proliferation and RAS-signaling in a cell line expressing the KRAS G12V mutation (H441 adenocarcinoma lung cancer cells).

Cell proliferation assay

H441 adenocarcinoma lung cancer cells were seeded at a density of 8000 cells per well in 96-well plates or 75000 cells per well in 24-well plates, cultured overnight and then treated with the doses of antibodies indicated in Table 9 below for 3 days. After the 3 days of incubation, cells were collected, and viable cells were determined by MTT (96-well plate studies) or trypan blue staining (24-well plate studies). The results are presented as the percentage of viable cells relative to that of the respective blank nanocapsules-treated controls.

Table 9: Inhibition of cell proliferation in H441 adenocarcinoma lung cancer cells expressing KRAS G12V mutation elicited by C16-HA, HA and PSA nanocapsules (NCs) prototypes (different doses/cell).

| Formulation | aG12V dose (ng/cell) | Individual experiment | Inhibition of cell proliferation vs. Blank NCs |
|---|---|---|---|
| C16-HA | 0.4 | #1 | 40% |
| | | #2 | 30% |
| | | #2 | 32% |
| | | #3 | 39% |
| PSA | 0.4 | #2 | 25% |
| HA | 0.27 | #4 | 35% * |
| | 0.4 | #4 | 54% * |
| aG12V control | 0.4 | #4 | 0% * |
| (*) 24-well plate studies | | | |

Western blotting. Cells were seeded in 6-well plates, cultured overnight, and then treated with C16-HA NCs (blank and anti-KRAS G12V mAb-loaded NCs at 166 nM mAb, 0.4 ng mAb/cell dose) during 20 h. Western blotting was performed using specific antibodies following standard procedures. For quantification of western blotting data, band intensities were quantified using ImageJ software and normalized to values of the loading control. The phosphorylation levels of proteins were normalized to the total levels of each protein, equivalently loaded on SDS-PAGE gels. Relative band intensity was expressed as the percent of reduction compared to the value of the corresponding control (blank nanocapsules-treated control). The scans of western blots are depicted in FIG. 4b, together with the corresponding inhibition of cell proliferation (FIG. 4a) obtained in an experiment performed in parallel.

FIG. 4. (A) Inhibition of cell proliferation and (B) reduction of ERK phosphorylation produced after incubation with C16-HA NCs (blank and anti-KRAS G12V mAb-loaded nanocapsules at 166 nM mAb, 0.4 ng mAb/cell dose) in H441 adenocarcinoma lung cancer cells expressing KRAS G12V mutation [C(-): untreated cells; BL: blank C16-HA NCs; aG12V NC: anti-KRAS G12V-loaded C16-HA NCs].

Conclusions: Anti-KRAS G12V mAb-loaded polymeric nanocapsules inhibited the *in vitro* growth of KRAS G12V mutant tumor cells in percentages ranging from 25 to >50% depending on the experimental conditions. Interference in the RAS signaling pathway was evidenced by a 30-40% reduction in ERK phosphorylation determined by Western Blotting.

**EXAMPLE 7: *In vivo* assays: efficacy of nanocapsules containing anti-KRAS G12V mAbs in reducing tumor growth in two mice models bearing KRAS G12V mutation: subcutaneous pancreatic xenograft and colon orthotopic tumor models**

This example illustrates the ability of polymeric anti-KRAS G12V mAb-loaded PSA-tLyp-1 nanocapsules in reducing the tumor growth in two different tumor mice models; a pancreatic subcutaneous xenograft tumor model and a colon orthotopic tumor model.

Pancreatic subcutaneous xenograft tumor model

A pancreatic xenograft tumor model was generated by inoculating subcutaneously PA-TU-8902 cells expressing KRAS G12V mutation into the right and left thigh of Rag2-/- mice (6-weeks old). When the tumor volume reached approximately 150-170 $mm^3$, mice were randomly assigned to treatment cohorts, and PSA-tLyp-1 NCs or a saline vehicle control were administered intraperitoneally every 3 days (PSA-tLyp-1 NCs at a 5 mg/kg anti-KRAS G12V mAb dose x 4 administrations; 120 μL/dose). Tumor volumes and body weight were recorded at regular intervals. At sacrifice, mice treated with anti-KRAS G12V mAb-loaded PSA-tLyp-1 NCs presented a percent tumor growth around 2-fold lower than the saline control (FIG. 5). No sign of toxicity was recorded in terms of bodyweight loss and animal behavior (data not shown).

Colon orthotopic tumor model

SW480 cell line-derived subcutaneous tumor fragments were implanted in the colon of Athymic Nude-Foxn1nu (5-weeks old) mice to generate a SW480 cell line-derived orthotopic xenograft model. Three weeks after implantation, mice were randomized and assigned to different groups in order to receive the different treatments. Anti-KRAS G12V mAb-loaded HA NCs at a mAb dose of 10 mg/kg were administered intravenously via the tail vein during a 3-weeks treatment period (week 1: 2 doses, i.e., 1 dose every 3 days; week 2: 2 doses, i.e, 1 dose every 3 days; week 3: 4 doses, i.e., 1 dose every 2 days) 150 μL/dose). PBS (control) was administered as control of non-treated animals at the same days. Final tumor weights after sacrifice are shown in FIG. 6. In order to quantify the extent of histologic regression changes, tumor slides were scanned and analyzed with the NDP. View2 viewing software (Hamamatsu), and the percentage of necrosis was calculated in every case. Results from 2 different tumors per treatment group are represented in FIG. 7. Mice behavior and weight were monitored during treatment to detect any sign of treatment toxicity (FIG. 8).

Conclusions: anti-KRAS G12V mAb-loaded polymeric nanocapsules significantly inhibited the tumor growth *in vivo* in two different KRAS G12V expressing cell line models: a pancreatic subcutaneous xenograft tumor model (PA-TU-8902 cells), and a colorectal orthotopic tumor model (SW480 cells), where very remarkable histologic regression changes in terms of tumor necrosis (>70%) were found at sacrifice. No bodyweight loss was registered during the 3-weeks study, thus showing no signs of treatment toxicity.

**REFERENCE LIST**

Non-patent literature:

Cox et al., 2014. Drugging the undruggable Ras: mission possible? Nat Rev Drug Discov. 2014 November; 13(11): 828-851

Shin et al., 2017. Antibody targeting intracellular oncogenic Ras mutants exerts anti-tumour effects after systemic administration. Nat Commun. 2017 May 10;8: 15090

Akishiba et al., 2017. Cytosolic antibody delivery by lipid-sensitive endosomolytic peptide. Nat Chem. 2017;9(8):751

Slastnikova et al., 2018. Targeted intracellular delivery of antibodies: The state of the art, Front. Pharmacol. 2018;9: 1208

Singh et al., 2019. Antibody delivery for intracellular targets: Emergent therapeutic potential, Bioconjugate Chem. 2019; 30: 1028

Carney et al., 1986. Monoclonal antibody specific for an activated RAS protein. Proc Natl Acad Sci U S A. 1986 Oct;83(19):7485-9

Römpp Lexikon Lacke und Druckfarben, Georg Thieme Verlag, Stuttgart, N.Y., 1998, "Hydrophilicity", "Hydrophobicity", pages 294 and 295.

Bertrand et al., 2014. Cancer Nanotechnology: The impact of passive and active targeting in the era of modern cancer biology, Advanced Drug Delivery Reviews 66 (2014) 2-25

Gilad et al., 2016. Recent innovations in peptide based targeted delivery to cancer cells, Biomedicines, 4 (2016)

Zhou et al., 2016. Aptamers: A promising chemical antibody for cancer therapy, Oncotarget, 7 (2016) 13446-13463

Kapp et al., 2017. A Comprehensive Evaluation of the Activity and Selectivity Profile of Ligands for RGD-binding Integrins. Sci. Rep.7, 39805 (2017)

Ruoslahti, 2017. Tumor penetrating peptides for improved drug delivery, Advanced Drug Delivery Reviews, Volumes 110-111 (2017) Pages 3-12

Zhang et al., 2016. Cell-penetrating peptides as noninvasive transmembrane vectors for the development of novel multifunctional drug-delivery systems, Journal of Controlled Release, Volume 229 (2016) pages 130-139

Regberg et al., 2012. Applications of cell-penetrating peptides for tumor targeting and future cancer therapies, Pharmaceuticals, 5 (2012) 991-1007

Liu P. et al., 2019. Targeting the untargetable KRAS in cancer therapy, Acta Pharm. Sin. B., 9 (2019) 871-879

Ferrer et al., 2018. KRAS-Mutant non-small cell lung cancer: From biology to therapy, Lung Cancer, 124 (2018) 53-64

Fisher et al., 2001. Induction and apoptotic regression of lung adenocarcinomas by regulation of a K-Ras transgene in the presence and absence of tumor suppressor genes, Genes and Development, 15 (2001) 3249-326

Ying et al., 2012. Oncogenic Kras Maintains Pancreatic Tumors through Regulation of Anabolic Glucose Metabolism, 149 (2012) 656-670

Lee and Mooney, 2012. Alginate: properties and biomedical applications. Prog Polym Sci., 37 (2012) 106-126

Patent literature:

EP0190033
US5084380
US5443956
WO2019086627A1
WO2012047317

## Claims

**1.** A composition, comprising:

a plurality of nanoentities comprising an inner core surrounded by an outer shell, the outer shell comprising a polymer, the inner core comprising at least one hydrophobic compound,
wherein the nanoentities comprise a pharmaceutical agent, wherein the pharmaceutical agent is an antibody or a

fragment thereof,
wherein the nanoentities are capable of intracellularly delivering the antibody or the fragment thereof, and wherein the antibody or the fragment thereof binds to an intracellular epitope of an activated mutated KRAS protein.

**2.** The composition according to claim 1, wherein the hydrophobic compound of the inner core comprises an oil, lipophilic surfactant, fatty acid, alkane, cycloalkane, bile salt, bile salt derivatives, terpenoid, terpene, terpene-derived moieties or lipophilic vitamin.

**3.** The composition according to claim 2, wherein the hydrophobic compound is an oil.

**4.** The composition according to any one of the preceding claims, wherein the antibody is a humanized antibody.

**5.** The composition according to any one of claims 1-4, wherein the epitope of the activated mutated KRAS protein comprises a mutation selected from the group consisting of the glycine residue at position 12, the glycine residue at position 13 or the glutamine residue at position 61 of the amino acid sequence of SEQ ID NO: 39.

**6.** The composition according to claim 5, wherein the mutation at position 12 of the amino acid sequence of SEQ ID NO: 39 is selected from the group consisting of arginine G12R, aspartic acid G12D, valine G12V and cysteine G12C.

**7.** The composition according to any one of claims 1-6, wherein the antibody or the fragment thereof binds to an epitope comprising a sequence selected from the group consisting of KLVWGAVGVGK SEQ ID NO: 40, KLVVVGADGVGK SEQ ID NO: 41, and KLVVVGACGVGK SEQ ID NO: 42.

**8.** The composition according to any one of claims 1-6, wherein the antibody or the fragment thereof is obtainable from a hybridoma cell line with a deposit number selected from the group consisting of DSM ACC3358, ATCC-HB-10083 D210, ATCC-HB-8698 DWP, ATCC-HB-10086-D113 and DSM ACC3359.

**9.** The composition according to any one of claims 1-6, wherein the antibody or the fragment thereof comprises a heavy-chain variable region CDR1: (SEQ ID NO: 33) SGYYWN, a heavy-chain variable region CDR2: (SEQ ID NO: 34) YIGYDGTNNYNPSLKN, a heavy-chain variable region CDR3: (SEQ ID NO: 35) LWDY, a light-chain variable region CDR1: (SEQ ID NO: 36) RSSQTIVHGNGNTYLE, a light-chain variable region CDR2: (SEQ ID NO: 37) TVSNRFS and a light-chain variable region CDR3: (SEQ ID NO: 38) FQGSHAPYT.

**10.** The composition according to any one of claims 1-6, wherein the antibody or the fragment thereof comprises a heavy-chain variable region CDR1: (SEQ ID NO: 45) SYYMY, a heavy-chain variable region CDR2: (SEQ ID NO: 46) EINPSNGGTNFNEKFKS, a heavy-chain variable region CDR3: (SEQ ID NO: 47) GGYGY, a light-chain variable region CDR1: (SEQ ID NO: 29) RSSKSLLYKDGKTYLN, a light-chain variable region CDR2: (SEQ ID NO: 30) LMSTRAS and a light-chain variable region CDR3: (SEQ ID NO: 31) QQVVEYPRT.

**11.** The composition according to any one of claims 1-10, wherein the polymer is selected from the group consisting of: polysialic acid (PSA), hyaluronic acid (HA), polyglutamic acid (PGA) and/or pegylated-polyglutamic acid (PGA-PEG), polylactic acid (PLA) and/or pegylated polylactic acid (PLA-PEG), poly(aspartic acid) (PASP) and/or pegylated-poly(aspartic acid) (PASP-PEG), poly(lactic-co-glycolic acid) (PLGA) and/or pegylated poly(lactic-co-glycolic acid) (PLA-PEG), alginic acid (ALG) and/or pegylated alginic acid (ALG-PEG), polymalic acid (PLMA) and/or pegylated polymalic acid (PLMA-PEG), and mixtures thereof.

**12.** The composition according to claim 11, wherein the polymer is PSA, HA or PGA-PEG.

**13.** The composition according to any one of claims 1-12, wherein the composition comprises a targeting moiety and/or a cell-penetrating peptide.

**14.** The composition according to claim 13, wherein the composition comprises a targeting moiety which is a CendR peptide selected from the group consisting of Lyp-1, tLyp-1, cLyp-1, and iRGD.

**15.** The composition according to any one of claims 1-14, wherein at least some of the polymers are linked to a hydrophobic moiety.

16. A composition according to any one of claims 1-15, for use as a medicament.

17. A composition according to any one of claims 1-15, for use in treating or preventing a disease associated with a mutation in a KRAS gene, wherein the disease is selected from the group consisting of cancer, Noonan syndrome (NS), Cardiofaciocutaneous syndrome (CFC), Costello syndrome, and epidermal nevus.

18. A method for preparing a composition as defined in any of one of claims 1-15, comprising the steps of:

a) preparing an aqueous phase comprising a polymer;
b) preparing a hydrophobic phase comprising a hydrophobic compound;
c) adding an antibody or a fragment thereof which binds to an intracellular epitope of an activated mutated KRAS protein to the aqueous phase or, optionally to the hydrophobic phase if the antibody or the fragment is highly concentrated; and
d) mixing the aqueous phase and the hydrophobic phase.

**Patentansprüche**

1. Eine Zusammensetzung, umfassend:

eine Vielzahl von Nanoentitäten, die einen inneren Kern umfassen, der von einer äußeren Hülle umgeben ist, wobei die äußere Hülle ein Polymer umfasst, wobei der innere Kern mindestens eine hydrophobe Verbindung umfasst, wobei die Nanoentitäten ein pharmazeutisches Mittel umfassen, wobei das pharmazeutische Mittel ein Antikörper oder ein Fragment davon ist,
wobei die Nanoentitäten in der Lage sind, den Antikörper oder das Fragment davon intrazellulär abzugeben, und wobei der Antikörper oder das Fragment davon an ein intrazelluläres Epitop eines aktivierten mutierten KRAS-Proteins bindet.

2. Die Zusammensetzung nach Anspruch 1, wobei die hydrophobe Verbindung des inneren Kerns ein Öl, ein lipophiles Tensid, eine Fettsäure, ein Alkan, ein Cycloalkan, ein Gallensalz, Gallensalzderivate, ein Terpenoid, ein Terpen, von Terpen abgeleitete Teile oder ein lipophiles Vitamin umfasst.

3. Die Zusammensetzung nach Anspruch 2, wobei die hydrophobe Verbindung ein Öl ist.

4. Die Zusammensetzung nach einem der vorhergehenden Ansprüche, wobei der Antikörper ein humanisierter Antikörper ist.

5. Die Zusammensetzung nach einem der Ansprüche 1 bis 4, wobei das Epitop des aktivierten mutierten KRAS-Proteins eine Mutation umfasst, die aus der Gruppe ausgewählt ist, die aus dem Glycinrest an Position 12, dem Glycinrest an Position 13 oder dem Glutaminrest an Position 61 der Aminosäuresequenz von SEQ ID Nr.: 39 besteht.

6. Die Zusammensetzung nach Anspruch 5, wobei die Mutation an Position 12 der Aminosäuresequenz von SEQ ID Nr.: 39 ausgewählt aus der Gruppe bestehend aus Arginin G12R, Asparaginsäure G12D, Valin G12V und Cystein G12C ist.

7. Die Zusammensetzung nach einem der Ansprüche 1 bis 6, wobei der Antikörper oder das Fragment davon an ein Epitop bindet, das eine Sequenz umfasst, die ausgewählt aus der Gruppe bestehend aus KLVVVGAVGVGK SEQ ID Nr.: 40, KLVVVGADGVGK SEQ ID Nr.: 41 und KLVVVGACGVGK SEQ ID Nr.: 42 ist.

8. Die Zusammensetzung nach einem der Ansprüche 1 bis 6, wobei der Antikörper oder das Fragment davon aus einer Hybridomzellinie mit einer Hinterlegungsnummer, die ausgewählt ist aus der Gruppe bestehend aus DSM ACC3358, ATCC-HB-10083 D210, ATCC-HB-8698 DWP, ATCC-HB-10086-D113 und DSM ACC3359 erhalten werden kann.

9. Die Zusammensetzung nach einem der Ansprüche 1 bis 6, wobei der Antikörper oder das Fragment davon Folgendes umfasst: eine variable Schwerkettenregion CDR1: (SEQ ID Nr.: 33) SGYYWN, eine variable Schwerkettenregion CDR2: (SEQ ID Nr.: 34) YIGYDGTNNYNPSLKN, eine variable Schwerkettenregion CDR3: (SEQ ID Nr.: 35) LWDY, eine variable Leichtkettenregion CDR1: (SEQ ID Nr.: 36) RSSQTIVHGNGNTYLE, eine variable Leichtkettenregion CDR2: (SEQ ID Nr.: 37) TVSNRFS und eine variable Leichtkettenregion CDR3: (SEQ ID Nr.: 38) FQGSHAPYT.

**10.** Die Zusammensetzung nach einem der Ansprüche 1 bis 6, wobei der Antikörper oder das Fragment davon Folgendes umfasst: eine variable Schwerkettenregion CDR1: (SEQ ID Nr.: 45) SYYMY, eine variable Schwerkettenregion CDR2: (SEQ ID Nr.: 46) EINPSNGGTNFNEKFKS, eine variable Schwerkettenregion CDR3: (SEQ ID Nr.: 47) GGYGY, eine variable Leichtkettenregion CDR1: (SEQ ID Nr.: 29) RSSKSLLYKDGKTYLN, eine variable Leichtkettenregion CDR2: (SEQ ID Nr.: 30) LMSTRAS und eine variable Leichtkettenregion CDR3: (SEQ ID Nr.: 31) QQVVEYPRT.

**11.** Die Zusammensetzung nach einem der Ansprüche 1 bis 10, wobei das Polymer ausgewählt ist aus der Gruppe bestehend aus: Polysialinsäure (PSA), Hyaluronsäure (HA), Polyglutaminsäure (PGA) und/oder pegylierter Polyglutaminsäure (PGA-PEG), Polylactid (PLA) und/oder pegyliertem Polylactid (PLA-PEG), Polyasparaginsäure (PASP) und/oder pegylierter Polyasparaginsäure (PASP-PEG), Poly(lactid-co-glycolid) (PLGA) und/oder pegyliertem Poly(lactid-co-glycolid) (PLA-PEG), Alginsäure (ALG) und/oder pegylierter Alginsäure (ALG-PEG), Polymalat (PLMA) und/oder pegyliertem Polymalat (PLMA-PEG) und Mischungen davon.

**12.** Die Zusammensetzung nach Anspruch 11, wobei das Polymer PSA, HA oder PGA-PEG ist.

**13.** Die Zusammensetzung nach einem der Ansprüche 1 bis 12, wobei die Zusammensetzung einen Targeting-Teil und/oder ein Zell-penetrierendes Peptid umfasst.

**14.** Die Zusammensetzung nach Anspruch 13, wobei die Zusammensetzung einen Targeting-Teil umfasst, der ein CendR-Peptid ist, das aus der Gruppe ausgewählt ist, die aus Lyp-1, tLyp-1, cLyp-1 und iRGD besteht.

**15.** Die Zusammensetzung nach einem der Ansprüche 1 bis 14, wobei mindestens einige der Polymere an einen hydrophoben Teil gebunden sind.

**16.** Eine Zusammensetzung nach einem der Ansprüche 1 bis 15 zur Verwendung als Medikament.

**17.** Eine Zusammensetzung nach einem der Ansprüche 1 bis 15 zur Verwendung bei der Behandlung oder Vorbeugung einer Krankheit, die mit einer Mutation in einem KRAS-Gen verbunden ist, wobei die Krankheit aus der Gruppe ausgewählt ist, die aus Krebs, Noonan-Syndrom (NS), cardiofaciocutanem Syndrom (CFC), Costello-Syndrom und epidermalem Nävus besteht.

**18.** Ein Verfahren zur Herstellung einer Zusammensetzung wie in einem der Ansprüche 1 bis 15 definiert, umfassend die folgenden Schritte:

a) Herstellen einer wässrigen Phase, die ein Polymer umfasst;
b) Herstellen einer hydrophoben Phase, die eine hydrophobe Verbindung umfasst;
c) Hinzufügen eines Antikörpers oder eines Fragments davon, das an ein intrazelluläres Epitop eines aktivierten mutierten KRAS-Proteins bindet, zu der wässrigen Phase oder wahlweise zu der hydrophoben Phase, wenn der Antikörper oder das Fragment hochkonzentriert ist; und
d) Mischen der wässrigen Phase und der hydrophoben Phase.

## Revendications

**1.** Une composition, comprenant :

une pluralité de nanoentités comprenant un noyau interne entouré d'une enveloppe externe, l'enveloppe externe comprenant un polymère, le noyau interne comprenant au moins un composé hydrophobe, dans laquelle les nanoentités comprennent un agent pharmaceutique, dans laquelle l'agent pharmaceutique est un anticorps ou une fraction de celui-ci,
dans laquelle les nanoentités sont capables de délivrer intracellulairement l'anticorps ou la fraction de celui-ci, et dans laquelle l'anticorps ou la fraction de celui-ci se lie à un épitope intracellulaire d'une protéine KRAS mutée activée.

**2.** La composition selon la revendication 1, dans laquelle le composé hydrophobe du noyau interne comprend une huile, un tensioactif lipophile, un acide gras, un alcane, un cycloalcane, un sel biliaire, des dérivés de sel biliaire, un terpénoïde, un terpène, des fractions dérivées de terpène ou une vitamine lipophile.

3. La composition selon la revendication 2, dans laquelle le composé hydrophobe est une huile.

4. La composition selon l'une quelconque des revendications précédentes, dans laquelle l'anticorps est un anticorps humanisé.

5. La composition selon l'une quelconque des revendications 1 à 4, dans laquelle l'épitope de la protéine KRAS mutée activée comprend une mutation choisie dans le groupe constitué du résidu de glycine en position 12, du résidu de glycine en position 13 ou du résidu de glutamine en position 61 de la séquence d'acides aminés de la SEQ ID n ° : 39.

6. La composition selon la revendication 5, dans laquelle la mutation en position 12 de la séquence d'acides aminés de la SEQ ID n ° : 39 est choisie dans le groupe constitué par arginine G12R, acide aspartique G12D, valine G12V et cystéine G12C.

7. La composition selon l'une quelconque des revendications 1 à 6, dans laquelle l'anticorps ou la fraction de celui-ci se lie à un épitope comprenant une séquence choisie dans le groupe constitué par KLVVVGAVGVGK SEQ ID n ° : 40, KLVVVGADGVGK SEQ ID n ° : 41, et KLVVVGACGVGK SEQ ID n ° : 42.

8. La composition selon l'une quelconque des revendications 1 à 6, dans laquelle l'anticorps ou la fraction de celui-ci peut être obtenu(e) à partir d'une lignée cellulaire d'hybridome avec un numéro de dépôt choisi dans le groupe constitué par DSM ACC3358, ATCC-HB-10083 D210, ATCC-HB-8698 DWP, ATCC-HB-10086-D113 et DSM ACC3359.

9. La composition selon l'une quelconque des revendications 1 à 6, dans laquelle l'anticorps ou la fraction de celui-ci comprend une région variable à chaîne lourde CDR1 : (SEQ ID n ° 33) SGYYWN, une région variable à chaîne lourde CDR2: (SEQ ID n ° 34) YIGYDGTNNYNPSLKN, une région variable à chaîne lourde CDR3 : (SEQ ID n ° 35) LWDY, une région variable à chaîne légère CDR1 : (SEQ ID n ° 36) RSSQTIVHGNGNTYLE, une région variable à chaîne légère CDR2 : (SEQ ID n ° 37) TVSNRFS et une région variable à chaîne légère CDR3 : (SEQ ID n ° 38) FQGSHAPYT.

10. La composition selon l'une quelconque des revendications 1 à 6, dans laquelle l'anticorps ou la fraction de celui-ci comprend une région variable à chaîne lourde CDR1 : (SEQ ID n ° 45) SYYMY, une région variable à chaîne lourde CDR2: (SEQ ID n ° 46) EINPSNGGTNFNEKFKS, une région variable à chaîne lourde CDR3 : (SEQ ID n ° 47) GGYGY, une région variable à chaîne légère CDR1 : (SEQ ID n ° 29) RSSKSLLYKDGKTYLN, une région variable à chaîne légère CDR2 : (SEQ ID n ° 30) LMSTRAS et une région variable à chaîne légère CDR3 : (SEQ ID n ° 31) QQVVEYPRT.

11. La composition selon l'une quelconque des revendications 1 à 10, dans laquelle le polymère est choisi dans le groupe constitué par : l'acide polysialique (PSA), l'acide hyaluronique (HA), l'acide polyglutamique (PGA) et/ou l'acide polyglutamique pégylé (PGA-PEG), l'acide polylactique (PLA) et/ou l'acide polylactique pégylé (PLA-PEG), l'acide poly(aspartique) (PASP) et/ou l'acide poly(aspartique) pégylé (PASP-PEG), l'acide poly(lactique-co-glycolique) (PLGA) et/ou l'acide poly(lactique-co-glycolique) pégylé (PLA-PEG), l'acide alginique (ALG) et/ou l'acide alginique pégylé (ALG-PEG), l'acide polymalique (PLMA) et/ou l'acide polymalique pégylé (PLMA-PEG), et des mélanges de ceux-ci.

12. La composition selon la revendication 11, dans laquelle le polymère est le PSA, le HA ou le PGA-PEG.

13. La composition selon l'une quelconque des revendications 1 à 12, dans laquelle la composition comprend une fraction de ciblage et/ou un peptide pénétrant dans les cellules.

14. La composition selon la revendication 13, dans laquelle la composition comprend une fraction de ciblage qui est un peptide CendR choisi dans le groupe constitué de Lyp-1, tLyp-1, cLyp-1 et iRGD.

15. La composition selon l'une quelconque des revendications 1 à 14, dans laquelle au moins certains des polymères sont liés à une fraction hydrophobe.

16. Une composition selon l'une quelconque des revendications 1 à 15, pour son utilisation comme médicament.

17. Une composition selon l'une quelconque des revendications 1 à 15, pour son utilisation dans le traitement ou la prévention d'une maladie associée à une mutation dans un gène KRAS, dans laquelle la maladie est choisie dans le

groupe constitué par le cancer, le syndrome de Noonan (NS), le syndrome cardio-facio-cutané (CFC), le syndrome de Costello et le naevus épidermique.

**18.** Un procédé de préparation d'une composition telle que définie dans l'une quelconque des revendications 1 à 15, comprenant les étapes consistant à :

a) préparer une phase aqueuse comprenant un polymère ;
b) préparer une phase hydrophobe comprenant un composé hydrophobe ;
c) ajouter un anticorps ou une fraction de celui-ci qui se lie à un épitope intracellulaire d'une protéine KRAS mutée activée à la phase aqueuse ou, éventuellement, à la phase hydrophobe si l'anticorps ou la fraction est fortement concentré(e) ; et
d) mélanger la phase aqueuse et la phase hydrophobe.

FIG. 1

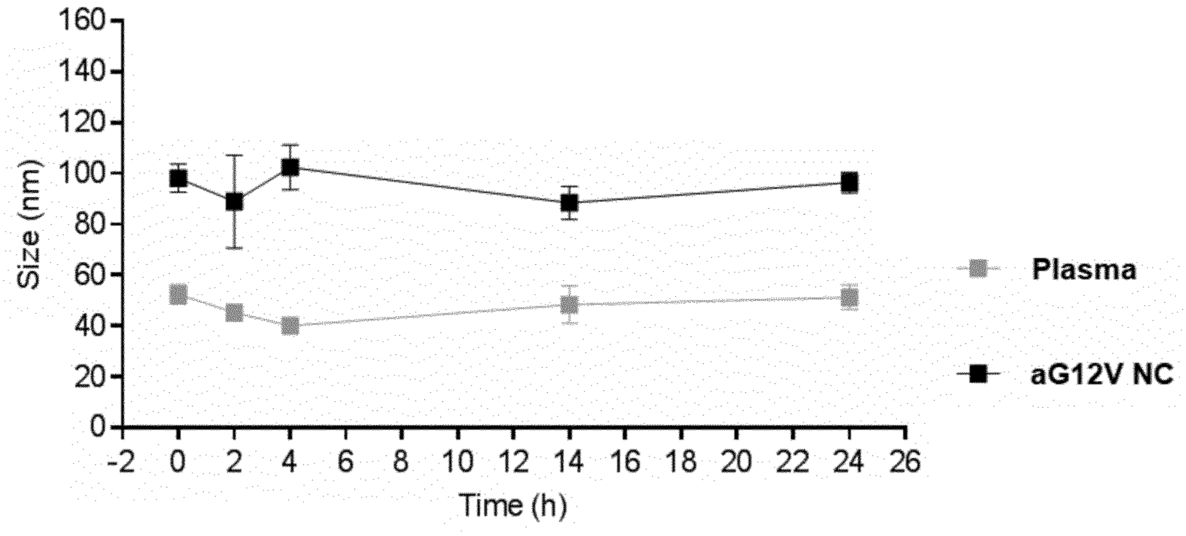
160
140
120
100
80
60
40
20
0
Size (nm)
-2
0
2
4
6
8
10
12
14
16
18
20
22
24
26
Time (h)
Plasma
aG12V NC

FIG. 2

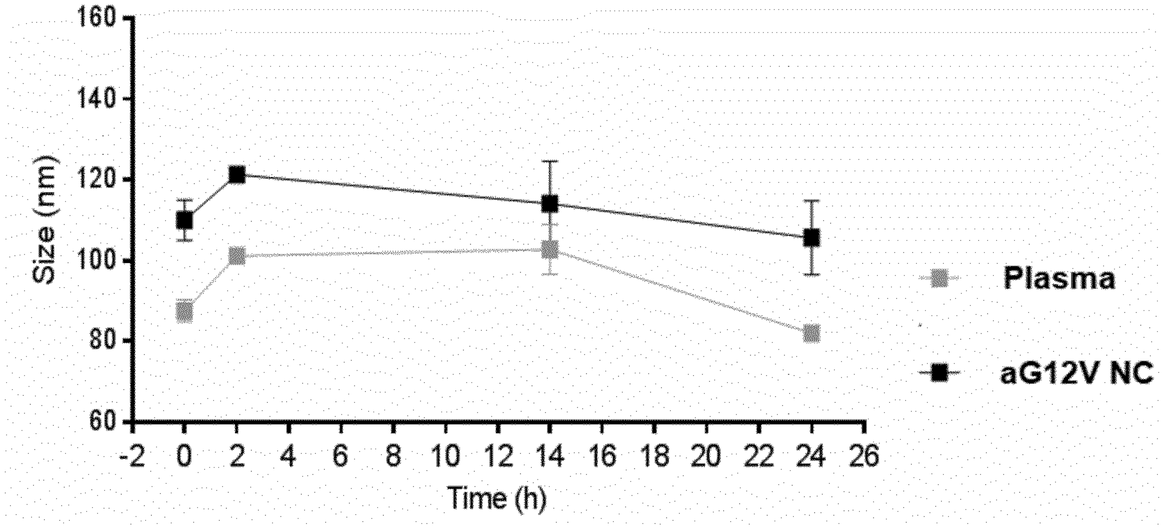
160
140
120
100
80
60
Size (nm)
-2
0
2
4
6
8
10
12
14
16
18
20
22
24
26
Time (h)
Plasma
aG12V NC

FIG. 3

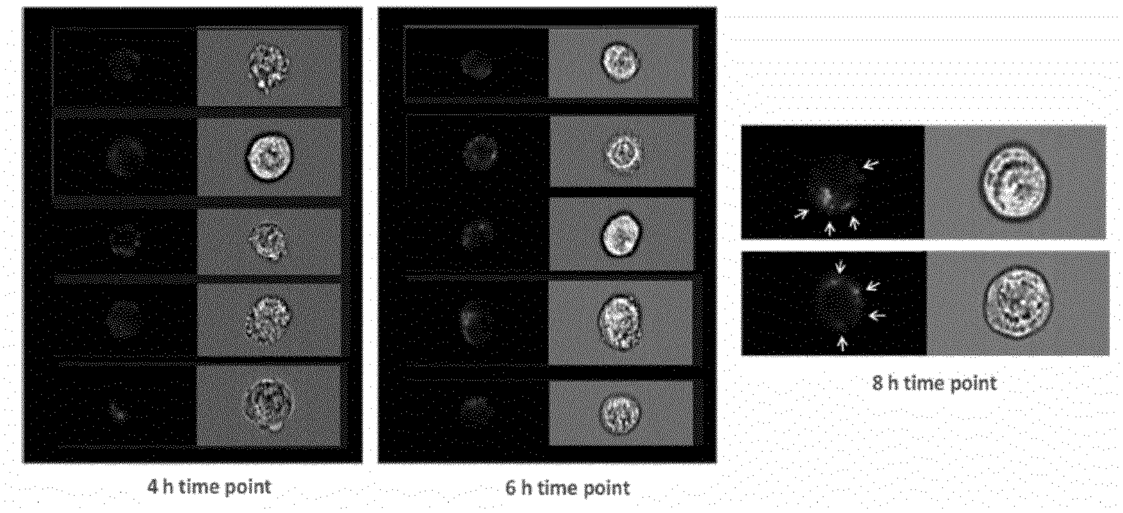

FIG. 4

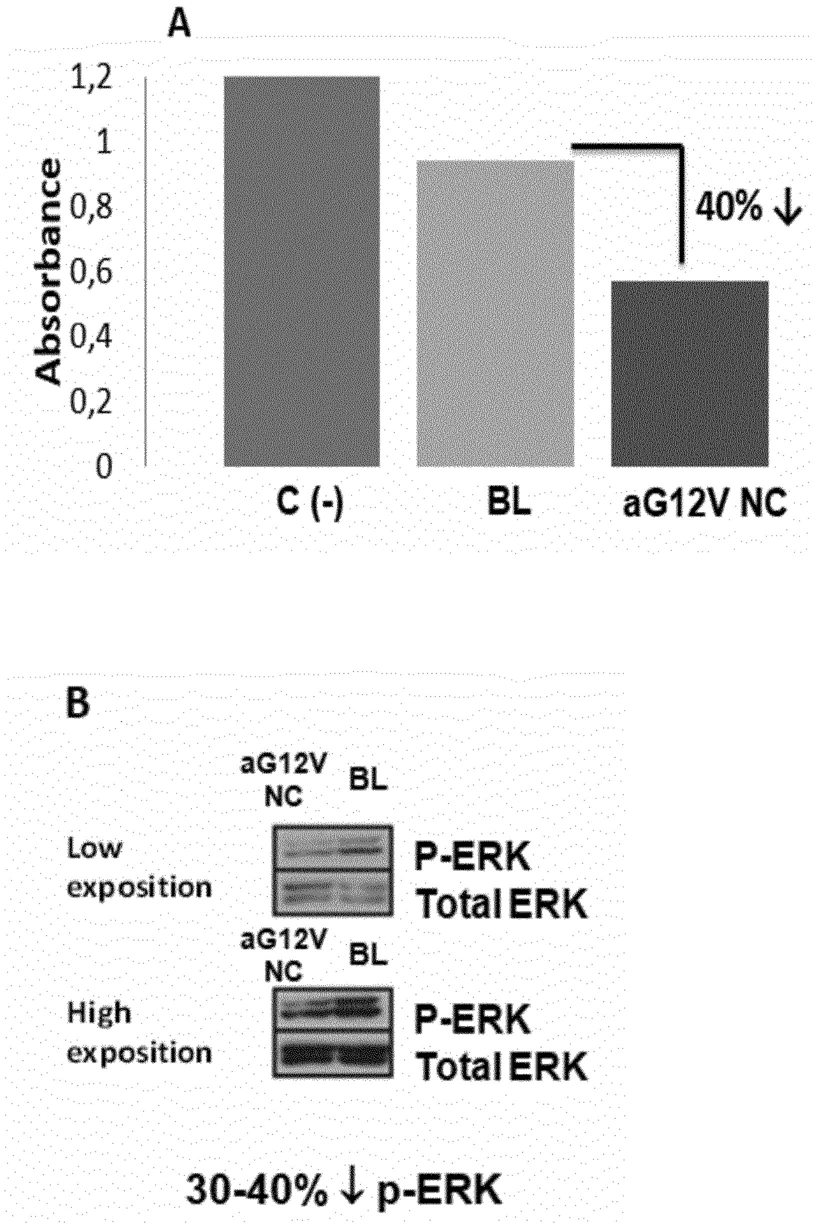
A
Absorbance
1,2
1
0,8
0,6
0,4
0,2
0
40% ↓
C (-)
BL
aG12V NC
B
aG12V NC
BL
Low exposition
P-ERK
Total ERK
aG12V NC
BL
High exposition
P-ERK
Total ERK
30-40% ↓ p-ERK

FIG. 5

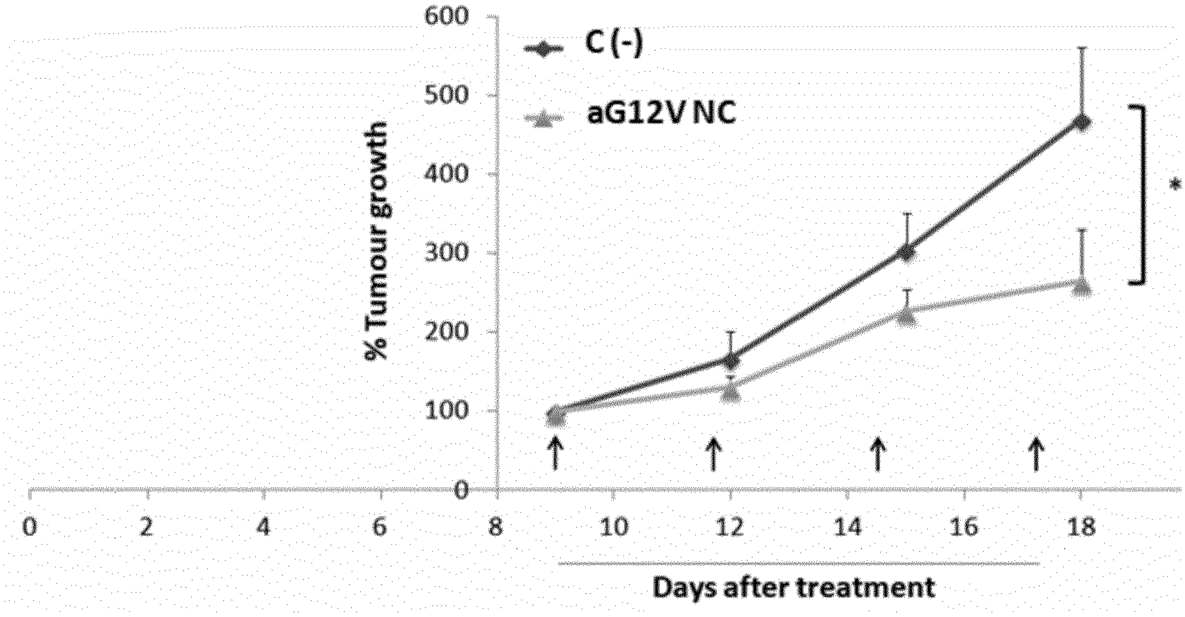
600
500
400
300
200
100
0
% Tumour growth
C (-)
aG12V NC
*
0
2
4
6
8
10
12
14
16
18
Days after treatment

FIG. 6

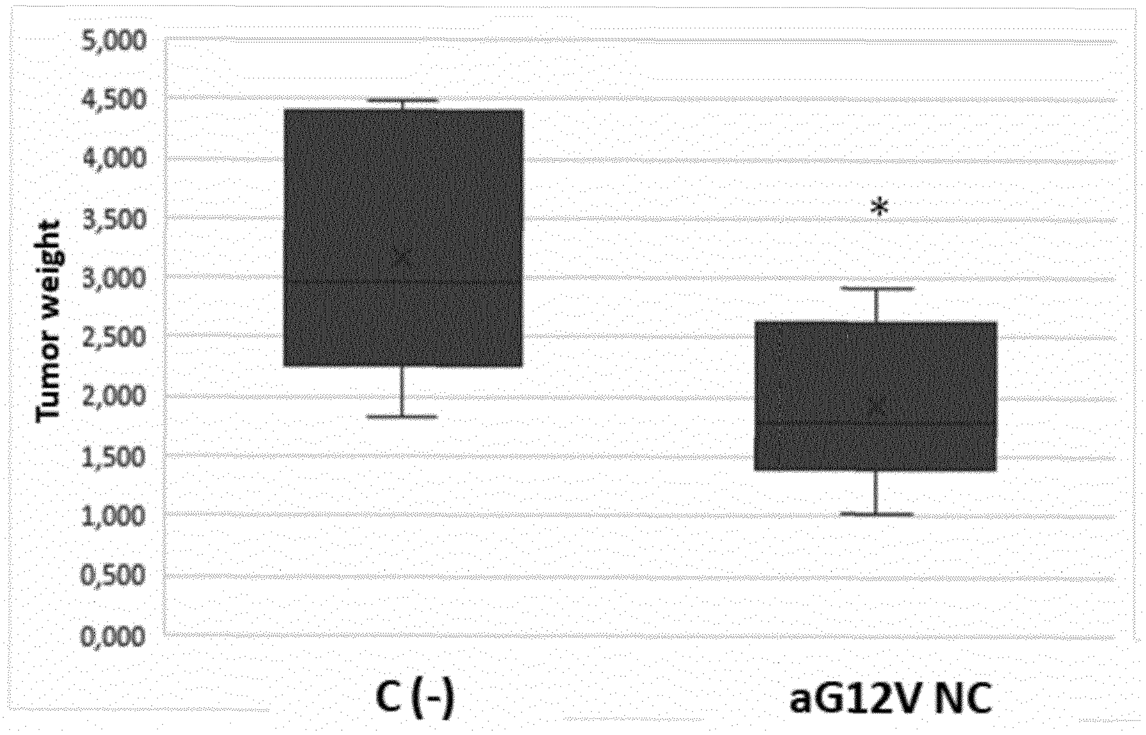
5,000
4,500
4,000
3,500
3,000
2,500
2,000
1,500
1,000
0,500
0,000
Tumor weight
*
C (-)
aG12V NC

FIG. 7

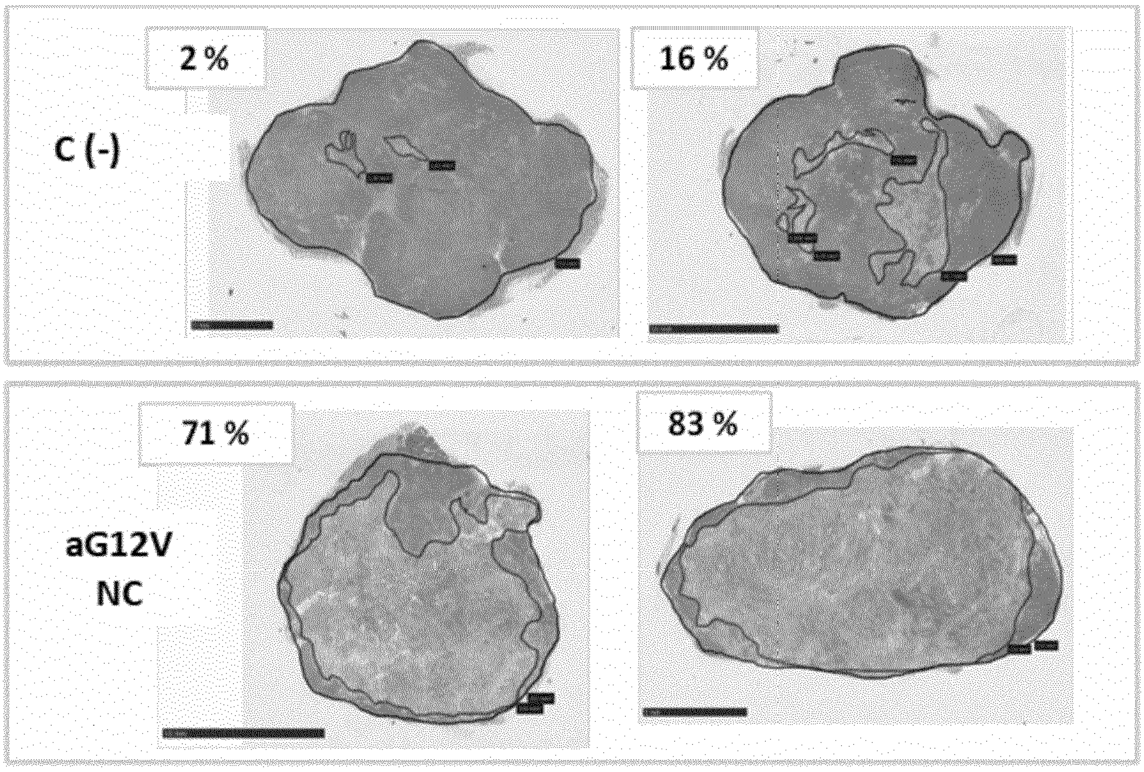

FIG. 8

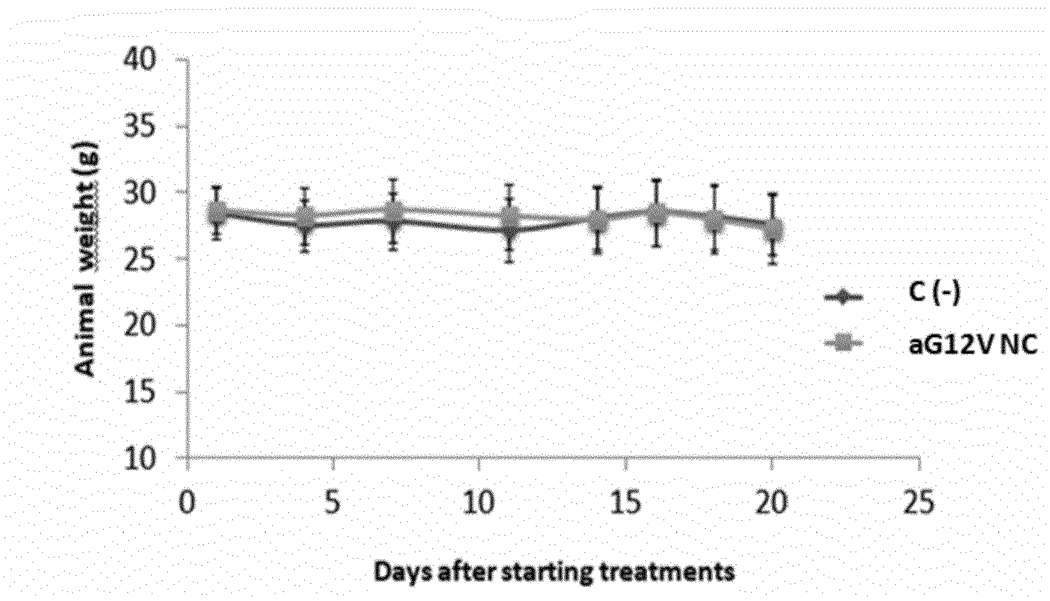
40
35
30
25
20
15
10
Animal weight (g)
0
5
10
15
20
25
Days after starting treatments
C (-)
aG12V NC

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 5084380 A
- EP 0190033 A
- US 5443956 A
- WO 2012047317 A
- WO 2019086627 A1


### Non-patent literature cited in the description


- Hydrophilicity'', ''Hydrophobicity. Römpp Lexikon Lacke und Druckfarben. Georg Thieme Verlag, 1998, 294, 295
- **COX et al.** Drugging the undruggable Ras: mission possible?. *Nat Rev Drug Discov.*, November 2014, vol. 13 (11), 828-851
- **SHIN et al.** Antibody targeting intracellular oncogenic Ras mutants exerts anti-tumour effects after systemic administration. *Nat Commun.*, 10 May 2017, vol. 8, 15090
- **AKISHIBA et al.** Cytosolic antibody delivery by lipid-sensitive endosomolytic peptide. *Nat Chem.*, 2017, vol. 9 (8), 751
- **SLASTNIKOVA et al.** Targeted intracellular delivery of antibodies: The state of the art. *Front. Pharmacol.*, 2018, vol. 9, 1208
- **SINGH et al.** Antibody delivery for intracellular targets: Emergent therapeutic potential. *Bioconjugate Chem.*, 2019, vol. 30, 1028
- **CARNEY et al.** Monoclonal antibody specific for an activated RAS protein. *Proc Natl Acad Sci U S A.*, October 1986, vol. 83 (19), 7485-9
- **BERTRAND et al.** Cancer Nanotechnology: The impact of passive and active targeting in the era of modern cancer biology. *Advanced Drug Delivery Reviews*, 2014, vol. 66, 2-25
- **GILAD et al.** Recent innovations in peptide based targeted delivery to cancer cells. *Biomedicines*, 2016, vol. 4
- **ZHOU et al.** Aptamers: A promising chemical antibody for cancer therapy. *Oncotarget*, 2016, vol. 7, 13446-13463
- **KAPP et al.** A Comprehensive Evaluation of the Activity and Selectivity Profile of Ligands for RGD-binding Integrins. *Sci. Rep.*, 2017, vol. 7, 39805
- **RUOSLAHTI**. Tumor penetrating peptides for improved drug delivery. *Advanced Drug Delivery Reviews*, 2017, vol. 110 (111), 3-12
- **ZHANG et al.** Cell-penetrating peptides as noninvasive transmembrane vectors for the development of novel multifunctional drug-delivery systems. *Journal of Controlled Release*, 2016, vol. 229, 130-139
- **REGBERG et al.** Applications of cell-penetrating peptides for tumor targeting and future cancer therapies. *Pharmaceuticals*, 2012, vol. 5, 991-1007
- **LIU P. et al.** Targeting the untargetable KRAS in cancer therapy. *Acta Pharm. Sin. B.*, 2019, vol. 9, 871-879
- **FERRER et al.** KRAS-Mutant non-small cell lung cancer: From biology to therapy. *Lung Cancer*, 2018, vol. 124, 53-64
- **FISHER et al.** Induction and apoptotic regression of lung adenocarcinomas by regulation of a K-Ras transgene in the presence and absence of tumor suppressor genes. *Genes and Development*, 2001, vol. 15, 3249-326
- **YING et al.** *Oncogenic Kras Maintains Pancreatic Tumors through Regulation of Anabolic Glucose Metabolism*, 2012, vol. 149, 656-670
- **LEE** ; **MOONEY**. Alginate: properties and biomedical applications. *Prog Polym Sci.*, 2012, vol. 37, 106-126